(19) Europäisches Patentamt
European Patent Office
Office européen des brevets

(11) Numéro de publication: **0 382 618 B1**

(12) # FASCICULE DE BREVET EUROPEEN

(45) Date de publication du fascicule du brevet: **20.09.95**

(51) Int. Cl.$^{6}$: **C07D 471/04**, C07D 307/82, C07D 209/30, A61K 31/435, A61K 31/34, A61K 31/40, //(C07D471/04,221:00,209:00)

(21) Numéro de dépôt: **90400306.8**

(22) Date de dépôt: **05.02.90**

Le dossier contient des informations techniques présentées postérieurement au dépôt de la demande et ne figurant pas dans le présent fascicule.

(54) **Dérivés aminoalkoxyphényle, leur procédé de préparation ainsi que les compositions en contenant.**

(30) Priorité: **06.02.89 US 306500**

(43) Date de publication de la demande:
**16.08.90 Bulletin 90/33**

(45) Mention de la délivrance du brevet:
**20.09.95 Bulletin 95/38**

(84) Etats contractants désignés:
**AT BE CH DE DK ES FR GB GR IT LI LU NL SE**

(56) Documents cités:
**EP-A- 0 235 111**
**EP-A- 0 302 793**
**US-A- 4 117 128**

**JOURNAL OF MEDICINAL CHEMISTRY, vol. 15, no. 5, 1972, pages 523-529, Columbus,Ohio, US; C.J. SHARPE et al.: "Basic ethers of 2-anilinobenzothiazoles and 2-anilinobenzoxazoles as potential antidepressants"**

(73) Titulaire: **SANOFI**
**32-34, rue Marbeuf**
**F-75008 Paris (FR)**
(84) Etats contractants désignés:
**CH DE DK ES FR GB GR IT LI LU NL SE AT**

(73) Titulaire: **S.A. SANOFI - LABAZ N.V.**
**Avenue De Béjar, 1**
**B-1120 Bruxelles (BE)**
(84) Etats contractants désignés:
**BE**

(72) Inventeur: **Gubin, Jean**
**Avenue des Citronniers, 24**
**B-1020 Bruxelles (BE)**
Inventeur: **Chatelain, Pierre**
**Avenue du Haras 111**
**B-1150 Bruxelles (BE)**

(74) Mandataire: **Le Guen, Gérard et al**
**CABINET LAVOIX**
**2, place d'Estienne d'Orves**
**F-75441 Paris Cédex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 382 618 B1

## Description

La présente invention se rapporte, d'une manière générale, à de nouveaux dérivés aminoalkoxyphényle ainsi qu'à leur procédé de préparation.

Plus particulièrement, les nouveaux dérivés aminoalkoxyphényle de l'invention peuvent être représentés par la formule générale :

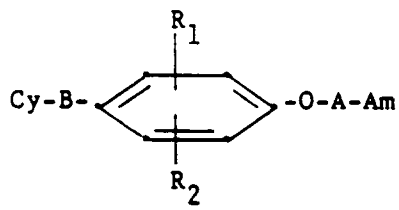

(1)

dans laquelle :

- B représente un groupement -S-, -SO- ou $-SO_2-$,
- $R_1$ et $R_2$, qui sont identiques ou différents, représentent chacun l'hydrogène, le radical méthyle ou éthyle ou un halogène tel que chlore, brome ou iode,
- A représente un radical alkylène, linéaire ou ramifié, ayant de 2 à 5 atomes de carbone ou le radical hydroxy-2 propylène dans lequel l'hydroxy est éventuellement substitué par un radical alkyle inférieur,
- Am représente un groupement :

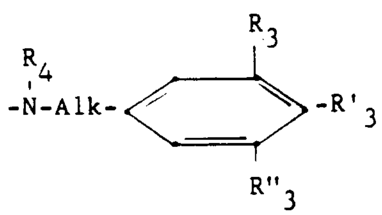

ou

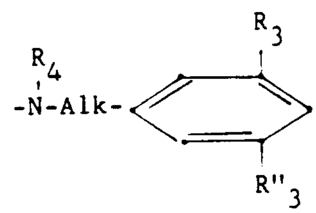

dans lequel : $R_3$, $R'_3$ et $R''_3$, qui sont identiques ou différents, représentent chacun un atome d'halogène tel que chlore ou brome, un groupement alkyle inférieur, un groupement alkoxy inférieur ou un groupement benzyloxy.

- $R_4$ représente l'hydrogène ou un radical alkyle,
- Alk représente une liaison simple ou un radical alkylène, linéaire ou ramifié ayant de 1 à 5 atomes de carbone,
- Cy représente un groupement de formule

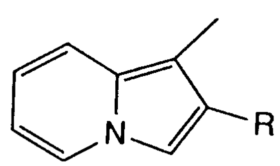

ou

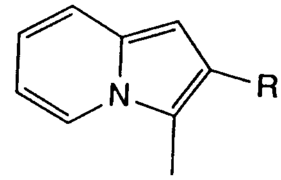

dans lequel

R représente l'hydrogène, un radical alkyle ayant jusqu'à huit atomes de carbone, un radical cycloalkyle en ($C_3$-$C_6$), un radical benzyle ou un radical phényle éventuellement substitué par un ou plusieurs substituants, qui peuvent être identiques ou différents, sélectionnés parmi des atomes d'halogène ou parmi des groupements alkyles ayant jusqu'à quatre atomes de carbone, alkoxy ayant jusqu'à quatre atomes de carbone ou nitro.

Dans le présent contexte, aussi bien dans la description que dans les revendications, les termes ci-dessous comportent les significations suivantes :
"alkyle" désigne les restes d'hydrocarbures, aliphatiques saturés linéaires ou ramifiés, ayant jusqu'à 8 atomes de carbone tels que méthyle, éthyle, n-propyle, isopropyle, n-butyle, isobutyle, tertiobutyle, n-pentyle, néopentyle, n-hexyle, n-heptyle ou n-octyle.
"alkyle inférieur" désigne les restes d'hydrocarbures saturés, linéaires ou ramifiês, ayant jusqu'à 4 atomes de carbone tel que méthyle, éthyle, n-propyle, isopropyle, n-butyle, isobutyle, tertiobutyle ou méthyl-1 propyle,
"alkoxy inférieur" désigne un groupement hydroxy substitué par un groupement alkyle inférieur tel que défini ci-dessus,
"cycloalkyle" désigne un groupement alicyclique ayant de 3 à 6 atomes de carbone, tels que cyclopropyle ou cyclohexyle.

Ainsi, en tenant compte des significations suivantes :

| | |
|---|---|
| R | représente, en particulier, un radical méthyle, éthyle, n-propyle, isopropyle, n-butyle, isobutyle, tertiobutyle, méthyl-1 propyle, n-pentyle, néopentyle, phényle, monofluoro-, monochloro-, ou monobromophényle, difluoro-, dichloro- ou dibromophényle, monométhyl- ou diméthylphényle, monométhoxy- ou diméthoxyphényle, un radical méthylphényle substitué par un atome d'halogène ou un radical cyclopropyle ou cyclohexyle, |
| $R_3$, $R'_3$ et $R''_3$ | représentent en particulier le radical méthyle ou méthoxy ou un atome de chlore, |
| $R_4$ | représente en particulier un radical méthyle, éthyle, n-propyle, isopropyle, n-butyle, isobutyle, tertiobutyle, n-pentyle, néopentyle, n-hexyle, n-heptyle ou n-octyle, |
| A | représente, en particulier, une chaine éthylène-1,2, propylène-1,3, méthyl-2 propylène-1,3, tétraméthylène-1,4 ou pentaméthylène-1,5, |

Une autre classe de composés de l'invention peut être représentée par la formule (1) dans laquelle $R_1$ et $R_2$ représentent chacun l'hydrogène.

Comme composés particulièrement intéressants de formule (1), on peut également citer ceux dans lesquels $R_3$, $R'_3$ et $R''_3$ représentent méthoxy.

D'autres composés intéressants de formule (1) sont ceux dans lesquels R représente le groupement isopropyle ou cyclopropyle.

L'invention se rapporte également aux sels pharmaceutiquement acceptables des composés de formule (1) formés à partir d'un acide organique ou inorganique.

Comme exemples de sels organiques de ce genre, on peut citer les oxalate, maléate, fumarate, méthanesulfonate, benzoate, ascorbate, pamoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate et théophylline acétate ainsi que les sels formés à partir d'un acide aminé tel que le sel de lysine ou d'histidine.

Comme exemples de sels inorganiques de ce genre, on peut citer les chlorhydrate, bromhydrate, sulfate, sulfamate, phosphate et nitrate.

Les composés de formule (1) peuvent exister dans certains cas sous la forme d'isomères optiques notamment en raison du carbone asymétrique présent lorsque A représente une chaîne hydroxy-2 propylène.

L'invention se rapporte à la fois à l'ensemble des isomères des composés de formule (1), isomères considérés sous forme dextrogyre ou lévogyre ou, sous forme de mélange, par exemple sous forme de mélange racémique.

On a trouvé que les composés de l'invention possèdent de remarquables propriétés pharmacologiques notamment des propriétés inhibitrices de la translocation calcique ainsi que des propriétés bradycardisantes, hypotensives et antiadrénergiques.

De ce point de vue, les composés préférés de l'invention sont ceux dans lesquels B représente un groupement $-SO_2-$.

Ces propriétés sont capables de rendre les composés en question très utiles dans le traitement de certains syndromes pathologiques du système cardiovasculaire en particulier dans le traitement de l'angine de poitrine, de l'hypertension, de l'arythmie, de l'insuffisance circulatoire cérébrale.

Dans le domaine antitumoral, les composés de l'invention pourront être utiles comme potentialisateurs d'anticancéreux.

Selon la voie d'administration choisie, la posologie journalière pour un être humain pesant 60 kg se situera entre 2 et 500 mg de principe actif.

De même, les composes de l'invention pourront être utilisés seuls ou en association avec un agent antiinflammatoire, pour réduire et/ou contrôler la pression intraoculaire excessive. A cet effet, les composés de l'invention pourront être utilisés pour le traitement d'affections pathologiques oculaires en particulier dans le traitement du glaucome.

Généralement, on administrera, à chaque oeil, de 5ng à 0,5mg de principe actif selon l'invention, la fréquence journalière d'administration dépendant de la gravité de l'affection à traiter.

En conséquence, l'invention se rapporte également à des compositions pharmaceutiques ou vétérinaires contenant, comme principe actif au moins un composé de formule (1) ou un sel pharmaceutiquement acceptable de ce dérivé en association avec un véhicule pharmaceutique ou un excipient approprié.

Les composés de l'invention peuvent être obtenus comme suit :

I. - Les composés de formule I dans laquelle B représente un groupement -S- ou $-SO_2-$ et A représente un radical alkylène peuvent être préparés, selon l'invention, en condensant, en présence d'un accepteur d'acide et dans un solvant polaire tel que le diméthylsulfoxyde ou un alcool par exemple le butanol, une cétone telle que la méthyl-éthyl-cétone, ou un solvant non polaire tel qu'un hydrocarbure aromatique, par exemple le benzène, le toluène ou un xylène, un dérivé alkoxy-4 phényle de formule générale :

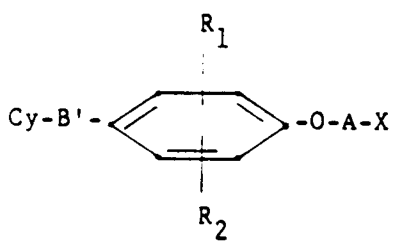

(2)

dans laquelle B' représente un groupement -S- ou $-SO_2-$, Cy, $R_1$ et $R_2$ ont la même signification que précédemment, A représente un radical alkylène tel que défini dans la formule (1) et X représente un atome d'halogène, de préférence le brome, ou un groupement alkylsulfonyloxy ayant de 1 à 4 atomes de carbone tel que par exemple méthanesulfonyloxy ou arylsulfonyloxy ayant de 6 à 10 atomes de carbone tel que benzènesulfonyloxy ou p-toluènesulfonyloxy, avec une amine de formule générale :

H-Am (3)

dans laquelle Am a la même signification que précédemment pour former le dérivé souhaité de formule (1) sous forme de base libre.

Généralement, la condensation en question est effectuée à une température comprise entre la température ambiante et la température de reflux du milieu, l'accepteur d'acide pouvant être par exemple un carbonate ou hydroxyde de métal alcalin ou un excès d'amine de formule (3).

Les composés de formule (2) en question peuvent être obtenus :

a) Lorsque X est un halogène, par condensation d'un dérivé hydroxy-4 phényle de formule générale :

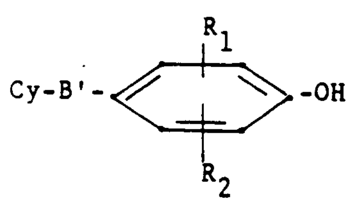

(4)

dans laquelle Cy, B', $R_1$ et $R_2$ ont la même signification que précédemment, avec un dihalogénoalcane de formule générale :

Hal-A-Hal (5)

dans laquelle A représente un radical alkylène tel que défini dans la formule (1) et Hal représente un atome d'halogène de préférence le brome et ce, au reflux, dans un solvant tel que la méthyl-éthyl-cétone ou le N,N-diméthylformamide et en présence d'un agent basique tel qu'un carbonate de métal

alcalin par exemple le carbonate de potassium, un hydrure de métal alcalin tel que l'hydrure de sodium, un hydroxyde de métal alcalin par exemple l'hydroxyde de sodium ou de potassium ou un alcoolate de métal alcalin par exemple le méthylate ou l'éthylate de sodium,

b) lorsque X représente un groupement alkylsulfonyloxy ou arylsulfonyloxy, par condensation d'un halogénure de formule générale :

Hal-W

dans laquelle Hal a la même signification que ci-dessus et W représente un radical alkylsulfonyle ayant de 1 à 4 atomes de carbone, par exemple méthanesulfonyle ou arylsulfonyle ayant de 6 à 10 atomes de carbone, par exemple benzènesulfonyle ou p-toluènesulfonyle, dans un solvant accepteur d'acide, par exemple la pyridine, avec un dérivé hydroxy alkoxy de formule générale :

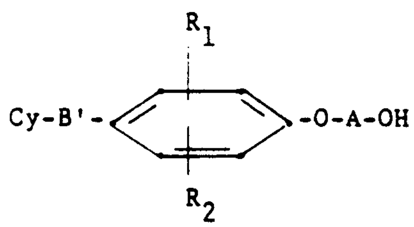

(6)

dans laquelle Cy, B', $R_1$ et $R_2$ ont la même signification que précédemment et A représente un radical alkylène tel que défini dans la formule (1).

Quant aux composés de formule (6), ceux-ci peuvent être préparés en condensant dans un solvant approprié tel que le N,N-diméthylformamide et en présence d'un agent basique tel qu'un carbonate de métal alcalin par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin par exemple le méthylate ou l'éthylate de sodium, un dérivé d'indolizine de formule (4) ci-dessus, avec un alcool halogéné de formule générale :

Hal-A-OH (7)

dans laquelle A représente un radical alkylène tel que défini dans la formule (1) et Hal a la même signification que précédemment.

Les amines de formule (3) sont des composés connus ayant été décrits dans des publications antérieures et pouvant être préparés selon des méthodes connues.

Certains composés de formule (4) sont des composés connus par exemple ceux dans lesquels Cy représente un groupement benzofuryle ou benzothiényle et B' représente un groupement $-SO_2-$ (brevet US N° 4.117.128) ou dans lesquels Cy représente un groupement indolizinyl-1 (demande EP N° 235.111).

Généralement, les autres composés de formule (4) peuvent être préparés en adaptant au composé désiré la méthode décrite dans le susdit brevet US ou les méthodes décrites ci-dessous.

Dans la plupart des cas, les composés de formule (4) peuvent être obtenus à partir d'un groupement benzènesulfonyle ou phénylthio, ce groupement étant O-protégé en position 4.

On fixe le groupement en question au carbocycle ou hétérocycle approprié en utilisant une réaction de Friedel-Crafts et on déprotège l'oxygène en position 4 du groupement benzènesulfonyle ou phénylthio au moyen de procédés classiques pour régénérer le groupement hydroxyle.

On a repris ci-dessous des exemples de procédés utilisés couramment pour préparer des dérivés de formule (4) :

1) Les composés de formule (4) dans laquelle Cy représente un groupement R-2 indolizinyl-3 peuvent être préparés en faisant réagir un dérivé d'indolizine de formule générale :

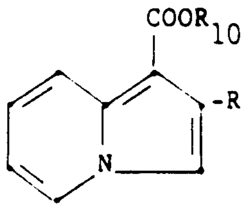

(8)

dans laquelle R a la même signification que précédemment et $R_{10}$ représente un radical alkyle inférieur de préférence éthyle, avec un halogénure de formule générale :

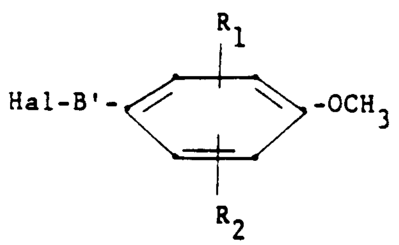

(9)

dans laquelle B', $R_1$, $R_2$ et Hal ont la même signification que précédemment et en présence d'un catalyseur de Friedel-Crafts tel que le chlorure d'aluminium, ce qui fournit un composé de formule générale :

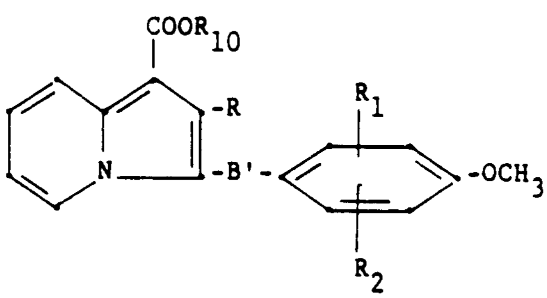

(10)

dans laquelle B', R, $R_1$, $R_2$ et $R_{10}$ ont la même signification que précédemment.
On déméthyle ensuite le composé de formule (10) au moyen d'un mélange éthanethiol/chlorure d'aluminium, pour obtenir le dérivé méthoxy-4 phénylé de formule générale :

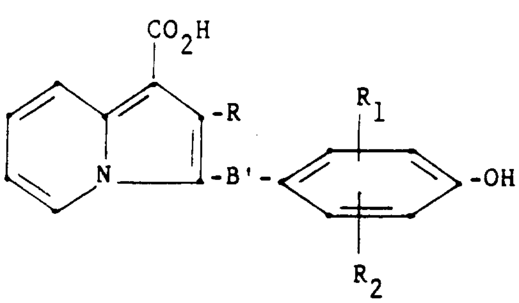

(11)

dans laquelle B', R, $R_1$ et $R_2$ ont la même signification que précédemment, dérivé que l'on chauffe aux environs de 200 °C ce qui fournit le composé souhaité de formule (4).
Les composés de formule (8) sont soit des composés connus ayant été publiés dans J. Chem. Soc. 1962 pp. 2627-2629 soit des composés qui peuvent être préparés selon la méthode y décrite.

Selon une autre méthode, on peut également obtenir les composés de formule (1) dans laquelle B représente un groupement -S- ou $SO_2$ et A représente un radical alkylène, de préférence ceux dans lesquels

A représente un radical propylène, en faisant réagir, en présence d'un agent basique tel qu'un carbonate de métal alcalin, par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin, par exemple le méthylate ou l'éthylate de sodium, un dérivé hydroxy-4 phényle de formule (4) ci-dessus avec un composé de formule générale :

X-A-Am (52)

dans laquelle X a la même signification que précédemment et représente de préférence le chlore ou un radical benzènesulfonyloxy ou p-toluènesulfonyloxy, A représente un radical alkylène et Am a la même signification que précédemment, la réaction ayant lieu à une température comprise entre la température ambiante et la température de reflux du milieu ainsi que dans un solvant polaire tel que la méthyl éthyl cétone ou le diméthylsulfoxyde pour former le dérivé aminoalkoxyphényle désiré de formule (1) sous forme de base libre.

Lorsque $R_4$ représente l'hydrogène, l'atome d'azote est de préférence protégé par un groupement labile par exemple un groupement protecteur que l'on peut éliminer en milieu basique par exemple le groupement tertiobutoxycarbonyle (BOC).

Les composés de formule (52) sont des composés connus ou qui peuvent être obtenus selon des procédés connus.

II. - Les composés de formule (1) dans laquelle B représente un groupement -SO-peuvent être obtenus en traitant, avec un agent oxydant, un sulfure de formule (1) dans laquelle B représente un groupement -S-, ce composé de formule (1) étant sous forme de base libre ou d'un sel de façon à obtenir le dérivé désiré de formule (1) sous forme de base libre ou de sel.

Lorsque le composé désiré est obtenu sous forme de sel, on peut régénérer la base libre par traitement avec un agent basique tel qu'un carbonate de métal alcalin par exemple le carbonate de potassium ou un bicarbonate de métal alcalin par exemple le bicarbonate de sodium.

Généralement, la réaction a lieu dans l'eau ou dans un solvant organique tel que le chlorure de méthylène et en présence d'un agent oxydant approprié tel que par exemple le périodate de sodium, le permanganate de potassium ou l'acide chloro-3 perbenzoïque.

Selon l'agent oxydant utilisé, on peut obtenir des mélanges de sulfoxydes ou de sulfones. Ces mélanges peuvent être séparés par des procédés conventionnels par exemple par chromatographie.

III. - Les composés de formule (1) dans laquelle B représente un groupement -S- ou $-SO_2-$ et A représente une chaîne hydroxy-2 propylène éventuellement substituée peuvent être obtenus en faisant réagir au reflux un dérivé hydroxy-4 phényle de formule (4) avec une épihalohydrine telle que l'épichlorhydrine ou l'épibromhydrine sous forme dextrogyre, lévogyre ou sous forme de mélange de ces isomères par exemple sous forme racémique et en présence d'un agent basique tel qu'un carbonate de métal alcalin par exemple le carbonate de potassium, un hydroxyde de métal alcalin par exemple l'hydroxyde de sodium ou de potassium, un hydrure de métal alcalin tel que l'hydrure de sodium ou un alcoolate de métal alcalin par exemple le méthylate ou l'éthylate de sodium et dans un solvant polaire telle que la méthyl-éthyl-cétone pour donner les dérivés oxyranylméthoxy de formule générale :

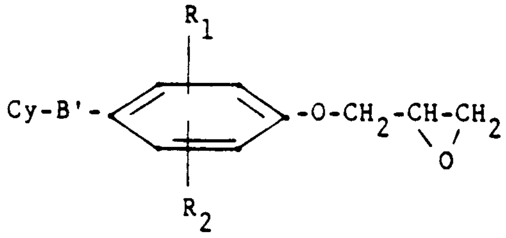

(57)

dans laquelle Cy, B', $R_1$ et $R_2$ ont la même signification que précédemment.

Les dérivés oxyranylméthoxy de formule (57) sont alors traités au reflux avec une amine de formule (3) et ce, dans un solvant polaire tel que la méthyl-éthyl-cétone ou dans un excès d'amine de formule (3) pour donner le dérivé désiré de formule (1) sous forme de base libre dans laquelle A représente une chaîne

hydroxy-2 propylène, dérivé que l'on peut faire réagir, si on le désire, avec un halogénure d'alkyle inférieur en présence d'une base forte pour former les composés de formule (1) sous forme de base libre dans laquelle A représente une chaîne hydroxy-2 propylène dans laquelle l'hydroxy est substitué par un radical alkyle inférieur.

Dans certains cas, des produits secondaires peuvent se former parallèlement aux composés de formule (57) ci-dessus, par exemple des dérivés (halogéno-3 hydroxy-2 propoxy)-4 benzènesulfonyles.

Par réaction avec l'amine de formule (3), ces dérivés donneront naissance néanmoins aux composés désirés de formule (1) dans laquelle A représente une chaîne hydroxy-2 propylène.

Les composés de formule (1) ainsi obtenus sous forme de base libre peuvent ensuite être transformés en sels pharmaceutiquement acceptables par réaction avec un acide organique ou inorganique approprié par exemple l'acide oxalique, maléique, fumarique, méthanesulfonique, benzoïque, ascorbique, pamoïque, succinique, hexamique, bisméthylènesalicylique, éthanedisulfonique, acétique, propionique, tartrique, salicylique, citraconique, aspartique, palmitique, stéarique, itaconique, glycolique, p-aminobenzoïque, glutamique, benzènesulfonique, théophylinne acétique , ou avec la lysine ou l'histidine.

La demande de brevet EP-A-0350 384 décrit des dérivés de sulfonyl-3-indolizine de formule

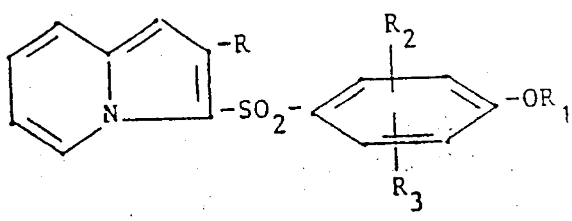

utilisables comme intermédiaires de synthèse.

On a décrit dans le brevet EP-A-235111 des dérivés phénylalkylaminoalkoxybenzènesulfonyl-1 indolizine, le groupement phényle étant substitué par un ou plusieurs substituants identiques ou différents sélectionnés parmi des atomes d'halogène, des groupements alkyles inférieurs ou des groupements alkoxy inférieurs.

Dans le cadre de cette définition, on n'a cependant cité et exemplifié spécifiquement que des dérivés phényle monosubstitués en positions 3 ou 4 ou disubstitués en positions 3 et 4 et plus précisément des dérivés méthoxy-3 ou -4 phényles ou diméthoxy-3,4 phényles.

On a maintenant trouvé, de manière tout à fait surprenante, que des composés analogues à ces dérivés phényl-3 ou -4 monosubstitués ou phényl-3,4 disubstitués en l'occurrence des dérivés phényl-3,5 disubstitués ou phényl-3,4,5 trisubstitués présentent des propriétés pharmacologiques particulièrement intéressantes, puisque supérieures à celles des dérivés phényles, mono- ou di-substitués en question.

Ainsi, on a trouvé que les composés de l'invention sont doués de propriétés inhibitrices de la translocation calcique et antiadrénergique $\alpha$ et $\beta$ généralement supérieures à celles des composés connus.

Par exemple, on a décelé chez les composés de l'invention un taux d'activité antiadrénergique analogue à celui des composés connus à des doses en moyenne dix fois inférieures à celles des composés connus.

En outre, on a trouvé de manière tout à fait inattendue que les composés de l'invention présentent <u>in vivo</u> un degré de métabolisation beaucoup moins rapide que celui des composés antérieurs.

Comme il a été rapporté en détail par R. CHARLIER dans "Bruxelles Médical", No. 9, septembre 1969, pages 543-560, il est admis qu'une médication antiangineuse doit être capable notamment d'antagoniser les réactions cardiovasculaires de type adrénergique. A cet effet, on a proposé des agents aptes à bloquer les récepteurs $\alpha$.

Cependant l'application clinique de tels composés au traitement de l'angor resta sans succès très probablement par le fait que les antagonistes des récepteurs $\alpha$ n'induisent qu'une neutralisation très partielle du système adrénergique, l'activité des récepteurs $\beta$ étant respectée.

Or, les manifestations hémodynamiques les plus indésirables qui surviennent chez l'angineux de poitrine au cours de ses accès douloureux sont avant tout cardiaques et relèvent de ce fait des récepteurs $\beta$.

Parallèlement, on a proposé des médications antagonistes des récepteurs adrénergiques $\beta$. Ces composés, d'un intérêt clinique réel, diminuent les crises d'angor en réduisant le travail cardiaque par ralentissement de la fréquence du rythme. Cependant, il n'y a pas de chute des résistances artérielles périphériques qui s'élèvent au contraire par libération du tonus $\alpha$.

Ces médications modifient toutefois certains paramètres hémodynamiques dans un sens qui, sur le plan fondamental, constitue une contre-partie défavorable à l'angineux de poitrine en particulier et au cardiaque en général.

Si l'on considère l'aspect antiadrénergique des β-bloquants, il devient évident que seules la tachycardie et l'augmentation de la puissance et de la vélocité de la contraction cardiaque sont susceptibles d'être neutralisées, l'hypertension artérielle relevant d'une stimulation des récepteurs α sur lesquels les antagonistes β n'ont pas d'action.

Or, si les perturbations cardiovasculaires entraînées par la stimulation des récepteurs β sont les plus préjudiciables à l'angineux, il n'en reste pas moins que l'hypertension artérielle joue également un rôle qui n'est pas négligeable.

Au surplus, le blocage des récepteurs β comporte un risque privant l'insuffisant cardiaque d'un mécanisme compensateur qu'il met normalement en jeu pour limiter son insuffisance circulatoire.

Ce mécanisme réflexe dont la composante principale emprunte la voie du système β-adrénergique aboutit notamment à une augmentation de la puissance et de la vélocité de la contraction cardiaque. Par conséquent , si ce système est bloqué, l'insuffisant cardiaque voit s'aggraver sa défaillance fonctionnelle. Il est donc logique de considérer que l'emploi d'un β-bloquant dont l'action est pure et complète comportera toujours un risque cardiaque.

Il paraît donc souhaitable de ne pas rechercher des propriétés antagonistes α ou β complètes, étant donné les effets secondaires qu'elles peuvent entraîner en clinique. Il semble plus logique de viser à amortir plutôt qu'à supprimer les perturbations cardiovasculaires qui caractérisent l'hyperstimulation du système adrénergique dans sa totalité.

Les composés de l'invention répondent à cet objectif puisqu'ils présentent des propriétés antiadrénergiques de type α et β incomplètes. Ils peuvent dont être considérés, non comme des β-bloquants mais comme des adrénofreinateurs c'est-à-dire des antagonistes partiels des réactions adrénergiques α et β potentiellement dépourvues des désavantages énumérés ci-dessus pour les β-bloquants.

En outre, la composante inhibitrice calcique mise en évidence chez les composés de l'invention complétera d'une manière remarquable leur spectre pharmacologique cardiovasculaire.

On sait, en effet, que la translocation des ions calcium est une des composantes essentielles du potentiel d'action au niveau des cellules cardiaques et que, à ce titre, elle joue un rôle primordial dans la conduction électrique ainsi que dans ses troubles éventuels (arythmie). En outre, il est connu que les ions calcium sont impliqués dans le couplage excitation-contraction lequel contrôle, au niveau de la musculature lisse le degré de vasoconstriction et, par la même occasion, joue un rôle critique dans la crise d'angine de poitrine.

Les composés antagonistes du calcium agissent au niveau de la membrane cellulaire en empêchant sélectivement le calcium d'intervenir dans le processus de contraction au sein de la cellule artérielle.

Or, il apparaît de plus en plus évident, à l'heure actuelle, que les résultats cliniques apportés par l'association d'inhibiteurs calciques et d'inhibiteurs β-adrénergiques sont meilleurs que lorsque chaque inhibiteur est utilisé isolément (J.A.M.A. 1982, 247, pages 1911-1917).

Il semble, en outre, qu'il n'existe, à l'heure actuelle, aucun β-bloquant exerçant en plus une action inhibitrice appréciable au niveau de la translocation calcique.

De ce point de vue, les composés de l'invention présentant à la fois une composante anticalcique et une composante antiadrénergique α et β seront d'un intérêt primordial car susceptibles d'applications thérapeutiques plus étendues qu'un β-bloquant isolé ou qu'un inhibiteur calcique isolé. A titre d'exemple, on citera :

- le [{[N-méthyl N-(triméthoxy-3,4,5 β-phénéthyl)amino]-3 propyl}oxy-4 benzènesulfonyl]-1 isopropyl-2 indolizine (SR 33827 ) (Ex. 1).
- le [{[N-méthyl N-(diméthoxy-3,5 β-phénéthyl) amino]-3 propyl}oxy-4 benzènesulfonyl]-1 isopropyl-2 indolizine (SR33918 )(Ex.12) [{[N-(Diméthoxy-3,5 β-phénéthyl)amino]-3 propyl}oxy-4 benzènesulfonyl]-1 isopropyl-2 indolizine (SR 33815)(Ex.11)

Cependant l'intérêt majeur de ces composés résidera dans le fait qu'ils pourront, grâce à leur composante anticalcique, être utilisés dans le traitement de l'angine au repos, syndrôme provoqué

par l'apparition d'un spasme au niveau des coronaires lequel est combattu actuellement par des composés tels que le diltiazem, le vérapamil ou la nifédipine.

Au surplus, des composés de l'invention se sont également montrés capables de provoquer une augmentation du débit coronaire de manière importante.

Les résultats de tests pharmacologiques effectués en vue de déterminer les propriétés cardiovasculaires des composés de l'invention sont répertoriés ci-dessous.

I. Propriétés inhibitrices calciques

Les propriétés inhibitrices de la translocation calcique au niveau membranaire présentées par les composés de l'invention ont été mises en évidence par mesure de leur action antagoniste vis-à-vis de la réponse contractile à la dépolarisation provoquée par le potassium sur l'aorte isolée de rat. Il est bien établi que la dépolarisation de la membrane d'un muscle lisse par le potassium rend cette dernière perméable au calcium extracellulaire et provoque la contraction musculaire.

Dès lors, la mesure de l'inhibition de la réponse contractile à la dépolarisation par le potassium ou la mesure d'un relâchement de la contraction tonique à la dépolarisation potassique peut constituer une évaluation de la puissance d'un composé en tant qu'inhibiteur de la perméabilité membranaire aux ions $Ca^{++}$.

La technique utilisée est la suivante :

Sur des rats mâles Wistar pesant environ 300g, on prélève l'aorte et on la coupe en bandelettes d'environ 40mm de longueur et 3mm de largeur.

On place ces fragments dans une cuve à organe isolé de 25ml contenant une solution de Krebs-bicarbonate modifiée (NaCl 112mM; KCl 5mM; $NaHCO_3$ 25mM; $KH_2PO_4$ 1mM; $MgSO_4$ 1,2mM; $CaCl_2$ 2,5 mM; glucose 11,5mM; eau distillée jusqu'à 1000ml) parcourue par un courant de carbogène et maintenue à 37°C. On relie la préparation à un microcapteur de force et on enregistre la réponse contractile après amplification sur un enregistreur.

On applique une tension de 2g à la préparation. On maintient celle-ci durant 60 minutes dans la solution de Krebs-bicarbonate modifiée puis on provoque les contractions en remplaçant la solution de Krebs-bicarbonate par une solution de Krebs-potassique (NaCl 17mM; KCl 100mM; $NaHCO_3$ 25mM; $KH_2PO_4$ 1mM; $MgSO_4$ 1,2 mM; $CaCl_2$ 2,5mM; glucose 11,5mM; eau distillée jusqu'à 1000ml). Lorsque la réponse contractile de la préparation est devenue reproductible, on introduit, dans le bain, une quantité donnée d'un composé de l'invention. Soixante minutes plus tard un nouveau spasme est provoqué par la dépolarisation potassique.

Les résultats obtenus sur l'aorte éprouvée sont alors exprimés en % de l'effet contracturant maximal avant l'incubation avec la substance à tester.

A titre d'exemples, les résultats suivants ont été obtenus, le composé de formule (1) étant sous la forme d'oxalate, de chlorhydrate ou de méthanesulfonate.

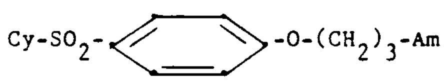

| Composé | Cy | Am | % de l'effet contracturant maximal. | | | |
|---|---|---|---|---|---|---|
| | | | | $10^{-8}M$ | $10^{-9}M$ | $10^{-10}M$ |
| Ex. 1 | 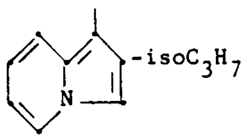 | 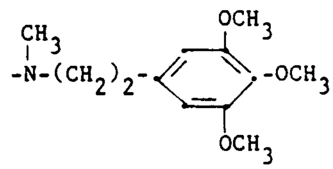 | | 21,4 | 65,7 | - |
| Ex. 2 | 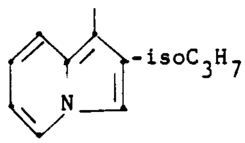 | 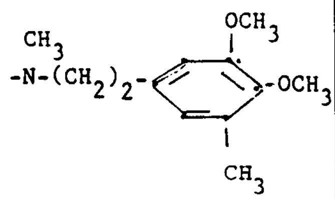 | | 21,1 | 58,5 | 78,9 |

| Composé | Cy | Am | % de l'effet contracturant maximal | | | |
|---|---|---|---|---|---|---|
| | | | | $10^{-8}$M | $10^{-9}$M | $10^{-10}$M |
| Ex. 5 | 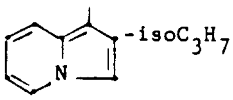 | 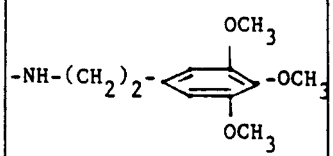 | | 49 | 89 | - |
| Ex.11 | 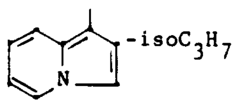 | 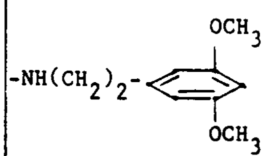 | | 30,3 | 73,4 | - |
| Ex.12 | 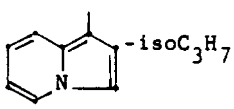 | 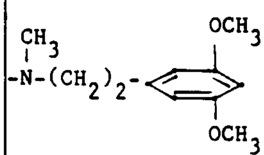 | | 21 | 57,7 | 81 |

Bz = benzyle

A titre de comparaison, on a obtenu les résultats suivants avec des composés connus :

| Composé | Cy | Am | % de l'effet contracturant maximal | | | |
|---|---|---|---|---|---|---|
| | | | | $10^{-8}M$ | $10^{-9}M$ | $10^{-10}M$ |
| A | 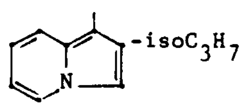 | 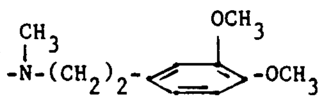 | | 40,1 | 81,7 | - |
| B | 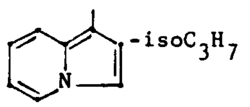 | 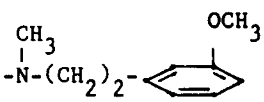 | | 49,5 | 78,1 | - |
| C | 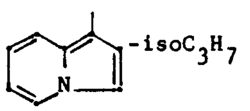 | 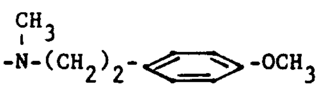 | | 69,6 | 86,5 | - |

II. Propriétés antiadrénergiques

Le but de ce test est de déterminer ta capacité des composés de l'invention de réduire l'augmentation de la pression sanguine induite par l'épinéphrine (effet anti-α) et l'accélération de la fréquence cardiaque induite par l'isoprénaline (effet anti-β) chez te chien préalablement anesthésié au pentobarbital et atropiné.

On détermine d'abord pour chaque chien la dose d'épinéphrine (entre 3 et 10 µg/kg) qui provoque une augmentation reproductible de la pression artérielle d'environ $133.10^2$Pa et la dose d'isoprénaline (1 à 2 µg/kg) qui provoque une augmentation reproductible de la fréquence cardiaque d'environ 70 battements/minute. On injecte alternativement toutes les dix minutes la dose d'épinéphrine et d'isoprénaline ainsi déterminée et après obtention de deux réponses de références successives, on administre une quantité du composé à étudier par voie intraveineuse.

- Effet anti-α

On enregistre le pourcentage de réduction de l'hypertension provoquée par le composé à étudier comparativement à l'hypertension de référence obtenue précédemment (environ 100 mm Hg).

-Effet anti-β

On enregistre le pourcentage de réduction de l'accélération de la fréquence cardiaque provoquée par le composé à étudier comparativement à la tachycardie de référence mesurée précédemment (environ 70 battements).

Dans les deux cas, on a exprimé les résultats de la réduction de la pression artérielle ou de la fréquence cardiaque comme suit :

+ pour une réduction < 50%

+ + pour une réduction ≧ 50%

+ + + pour une réduction sub-totale (réduction presque complète)

On a enregistré les résultats suivants :

| Composé | Dose (mg/kg) | Effet anti-$\alpha$ | Effet anti-$\beta$ |
|---|---|---|---|
| Ex. 1 | 0,055 | + + + | + + + |
| Ex. 11 | 0,027 | + + | + |
| | 0,055 | + + + | + + + |
| Ex. 12 | 0,033 | + + + | + + |

A titre comparatif, on a obtenu les résultats suivants avec des composés connus :

| Composé | Dose (mg/kg) | Effet anti-$\alpha$ | Effet anti-$\beta$ |
|---|---|---|---|
| A | 0,1 | + + + | + + + |
| B | 0,1 | + + + | + + + |
| C | 0,3 | + + | + |

III - Toxicité

La toxicité des composés de l'invention s'est révélée compatible avec leur emploi en thérapeutique.

Les compositions thérapeutiques selon l'invention peuvent être présentées sous toute forme convenant à l'administration en thérapie humaine ou vétérinaire. Pour ce qui concerne l'unité d'administration, celle-ci peut prendre la forme, par exemple, d'un comprimé, d'une dragée, d'une capsule, d'une gélule, d'une poudre, d'une suspension ou d'un sirop pour l'administration orale, d'un suppositoire pour l'administration rectale ou d'une solution ou suspension pour l'administration parentérale.

Les compositions thérapeutiques de l'invention pourront comprendre, par unité d'administration, par exemple de 50 à 500mg en poids d'ingrédient actif pour l'administration orale, de 50 à 200mg d'ingrédient actif pour l'administration rectale et de 50 à 150mg d'ingrédient actif pour l'administration parentérale.

Suivant la voie d'administration choisie, les compositions thérapeutiques ou vétérinaires de l'invention seront préparées en associant au moins un des composés de formule (1) ou un sel d'addition non toxique de ce composé avec un excipient approprié, ce dernier pouvant être constitué par exemple d'au moins un ingrédient sélectionné parmi les substances suivantes : lactose, amidons, talc, stéarate de magnésium, polyvinylpyrrolidone, acide alginique, silice colloidale, eau distillée, alcool benzylique ou agents édulcorants.

EXEMPLE 1

Préparation de l'oxalate acide de [{[N-méthyl N-(triméthoxy-3,4,5 $\beta$-phénéthyl)amino]-3 propyl} oxy-4 benzènesulfonyl]-1 isopropyl-2 indolizine (SR 33827A)

a) [(Bromo-3 propoxy)-4 benzènesulfonyl]-1 isopropyl-2 indolizine

On mélange 3,15g (0,01 mole) d'(hydroxy-4 benzènesulfonyl)-1 isopropyl-2 indolizine, 40,38g (0,2 mole; 20,3 ml) de dibromo-1,3 propane, 1,66g (0,012 mole) de carbonate de potassium et 20ml de N,N-diméthylformamide. On chauffe à 100°C et on suit la réaction par chromatographie sur couche mince (solvant : dichlorométhane/acétate d'éthyle 95/5). On laisse réagir durant 50 minutes, puis on élimine l'excès de dibromo-1,3 propane par évaporation sous pression réduite. On reprend dans l'acétate d'éthyle et on lave avec de l'hydroxyde de sodium dilué puis avec de l'eau. On sèche sur carbonate de potassium puis on filtre. On coule dans de l'eau, on extrait avec de l'acétate d'éthyle puis on lave avec de l'eau et avec une solution saturée de chlorure de sodium. On sèche sur sulfate de sodium et on concentre.

De cette manière, on obtient environ 4g de [(bromo-3 propoxy)-4 benzènesulfonyl]-1 isopropyl-2 indolizine sous forme brute que l'on recristallise dans un mélange acétate d'éthyle/hexane.

Rendement après recristallisation : 67%

P.F. : 133,4°C.

b) Oxalate acide de [{[N-méthyl N-(triméthoxy-3,4,5 β-phénéthyl)amino]-3 propyl} oxy-4 benzènesulfonyl]-1 isopropyl-2 indolizine

On mélange, à température ambiante 0,0025 mole de [(bromo-3 propoxy)-4 benzènesulfonyl]-1 isopropyl-2 indolizine, 0,005 mode de N-méthyl N-triméthoxy-3,4,5 β-phénéthylamine et 0,010 mode de carbonate de potassium dans 5ml de diméthylsulfoxyde. On agite le mélange pendant 22 heures, tout en suivant l'évolution de la réaction par chromatographie sur couche mince (solvant : méthanol), puis on verse le produit réactionnel dans l'eau.

On extrait avec du dichlorométhane et on lave avec une solution aqueuse saturée de chlorure de sodium. On sèche sur sulfate de sodium et on concentre pour obtenir environ 1,6g de produit brut. On purifie par chromatographie sur gel de silice en éluant avec un mélange acétate d'éthyle/méthanol 80/20.

On récupère ainsi la [{[N-méthyl N-(triméthoxy-3,4,5 β-phénéthyl) amino]-3 propyl} oxy-4 benzènesulfonyl]-1 isopropyl-2 indolizine sous forme de base libre.

On fait alors réagir 0,0018 mole de la base ainsi obtenue, avec 0,0018 mole d'acide oxalique dans un mélange acétate d'éthyle/éther éthylique.

De cette manière, on récupère l'oxalate acide de [{[N-méthyl N-(triméthoxy-3,4,5 β-phénéthyl)amino]-3 propyl} oxy-4 benzènesulfonyl]-1 isopropyl-2 indolizine que l'on peut recristalliser par exemple dans le méthanol.

P.F. : 182-183 °C (méthanol)

EXEMPLE 2

Préparation de l'oxalate acide d'isopropyl-2 [{[N-méthyl N-(diméthoxy-3,4 méthyl-5 β-phénéthyl)amino]-3 propyl} oxy-4 benzènesulfonyl]-1 indolizine (SR 33900A).

On agite pendant 3 jours un mélange de 0,0126 mole d'isopropyl-2 (hydroxy-4 benzènesulfonyl)-1 indolizine et 0,0189 mole de chloro-1 N-méthyl N-(diméthoxy-3,4 méthyl-5 β-phénéthyl)amino]propane dans 60ml de diméthylsulfoxyde anhydre en présence de 0,441 mode de carbonate de potassium. Après réaction, on verse le mélange dans un grand volume d'eau et on extrait avec 3 fois 100ml de toluène. On lave à l'eau, sèche sur sulfate de sodium, filtre et évapore à sec sous vide. On agite dans de l'heptane et on recristallise, dans l'éthanol, le produit formé. On obtient ainsi le produit désiré sous forme de base libre dont on forme l'oxalate dans l'acétate d'éthyle à l'ébullition.

De cette manière, on recueille l'oxalate acide d'isopropyl-2 [{[-méthyl N-(diméthoxy-3,4 méthyl-5 β-phénéthyl)amino]-3 propyl} oxy-4 benzène-sulfonyl]-1 isopropyl-2 indolizine.

P.F. : 189-190 °C (méthanol/éther éthylique)

De manière analogue à celle décrite précédemment, on a préparé les composés suivants :

Chlorhydrate de [{[N-méthyl N-(triméthoxy-3,4,5 β-phénéthyl)amino]-3 propyl} oxy-4 benzènesulfonyl]-1 isopropyl-2 indolizine (SR 33827 B) (Exemple 3)

P.F. 90 à 110 °C (pâteux)

Oxalate acide de [{[N-(méthoxy-3 méthyl-5 benzyloxy-4 β-phénéthyl)amino]-3 propyl} oxy-4 benzènesulfonyl]-1 isopropyl-2 indolizine (SR 33964 A) (Exemple 4)

P.F. : 165-167 °C

Oxalate acide de [{[N-diméthoxy-3,4 méthyl-5 β-phénéthyl)amino]-3 propyl} oxy-4 benzènesulfonyl]-1 isopropyl-2 indolizine (SR33969 A) (Exemple 5)

P.F. : 180-183 °C (acétate d'éthyle/méthanol)

Chlorhydrate de [{[N-(méthoxy-4 méthyl-5 benzyloxy-3 β-phénéthyl)amino]-3 propyl} oxy-4 benzènesulfonyl]-1 isopropyl-2 indolizine (SR 33979 A) (Exemple 6)

P.F. : 209-210 °C (acétate d'éthyle/méthanol)

Oxalate acide de [{[ N-(diméthoxy-3,5 β-phénéthyl)amino]-3 propyl} oxy-4 benzènesulfonyl]-1 isopropyl-2 indolizine (SR 33815A) (Exemple 11)

P.F. : 196-197 °C

Oxalate acide de [{[N-méthyl N-(diméthoxy-3,5 β-phénéthyl)amino]-3 propyl} oxy-4 benzènesulfonyl]-1 isopropyl-2 indolizine (SR 33918 A) (Exemple 12) P.F. : 169-170 °C.

## Revendications

### Revendications pour les Etats contractants suivants : AT, BE, CH, DE, DK, FR, GB, IT, LI, LU, NL, SE

**1.** Dérives aminoalkoxyphényles correspondant à la formule générale :

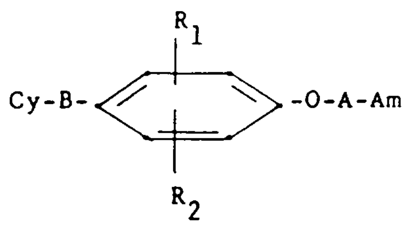

(1)

ainsi que leurs sels pharmaceutiquement acceptables dans laquelle :

| | |
|---|---|
| B | représente un groupement -S-, -SO- ou $-SO_2-$, |
| $R_1$ et $R_2$, | qui sont identiques ou différents, représentent chacun l'hydrogène, le radical méthyle ou éthyle ou un halogène tel que chlore, brome ou iode, |
| A | représente un radical alkylène, linéaire ou ramifié, ayant de 2 à 5 atomes de carbone ou le radical hydroxy-2 propylène dans lequel l'hydroxy est éventuellement substitué par un radical alkyle ayant jusqu'à quatre atomes de carbone, |
| Am | représente un groupement : |

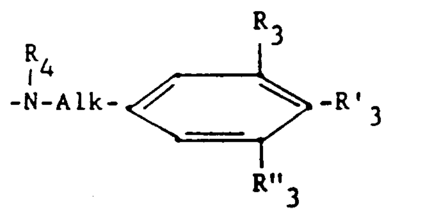

ou

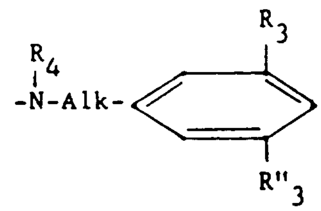

dans lequel : $R_3$, $R'_3$ et $R''_3$, qui sont identiques ou différents, représentent chacun un atome d'halogène tel que chlore ou brome, un groupement alkyle ayant jusqu'à quatre atomes de carbone ou un groupement alkoxy ayant jusqu'à quatre atomes de carbone, ou un groupe benzyloxy,

| | |
|---|---|
| $R_4$ | représente l'hydrogène ou un radical alkyle ayant jusqu'à huit atomes de carbone, |
| Alk | représente une liaison simple ou un radical alkylène, linéaire ou ramifié ayant de 1 à 5 atomes de carbone, |
| Cy | représente un groupement de formule |

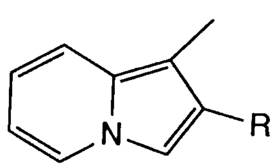

ou

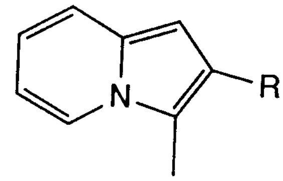

dans lequel

R représente l'hydrogène, un radical alkyle ayant jusqu'à huit atomes de carbone, un radical cycloalkyle en ($C_3$-$C_6$), un radical benzyle ou un radical phényle éventuellement substitué par un ou plusieurs substituants, qui peuvent être identiques ou différents, sélectionnés parmi des atomes d'halogène ou parmi des groupements alkyles ayant jusqu'à quatre atomes de carbone, alkoxy ayant jusqu'à quatre atomes de carbone ou nitro.

**2.** Dérivés aminoalkoxyphényles selon la Revendication 1 dans laquelle B représente un groupement $-SO_2-$.

**3.** Dérivés aminoalkoxyphényles selon la Revendication 1 dans laquelle $R_1$ et $R_2$ représentent chacun l'hydrogène.

**4.** Dérivés aminoalkoxyphényles selon la Revendication 1 dans laquelle $R_3$, $R'_3$ et $R''_3$ représentent méthoxy.

**5.** Dérivés aminoalkoxyphényles selon la Revendication 1 dans laquelle R représente le groupement isopropyle ou cyclopropyle.

**6.** Dérivés aminoalkoxyphényles selon la Revendication 1 dans laquelle le sel pharmaceutiquement acceptable est l'oxalate, le chlorhydrate ou le méthanesulfonate.

**7.** Dérivés d'aminoalkylkoxyphényles selon la revendication 1, choisis parmi :
la [{[N-méthyl N-(triméthoxy-3,4,5 $\beta$-phénéthyl)amino]-3propyl} oxy-4 benzènesulfonyl]-1 isopropyl-2 indolizine,
la [{[N-méthyl N-(diméthoxy-3,4 méthyl-5 $\beta$-phénéthyl)amino]-3 propyl} oxy-4 benzènesulfonyl]-1 isopropyl-2 indolizine,
la [{[N-diméthoxy-3,5 $\beta$-phénéthyl)amino]-3 propyl} oxy-4 benzènesulfonyl]-1 isopropyl-2 indolizine et leurs sels pharmaceutiquement acceptables.

**8.** Procédé de préparation de dérivés d'aminoalkoxyphényles selon la Revendication 1 dans laquelle A représente un radical alkylène et B représente un groupement -S- ou $-SO_2-$, caractérisé en ce que l'on condense, en présence d'un accepteur d'acide, dans un solvant polaire ou non polaire et à une température comprise entre la température ambiante et la température de reflux, un dérivé alcoxy-4 phényle de formule générale :

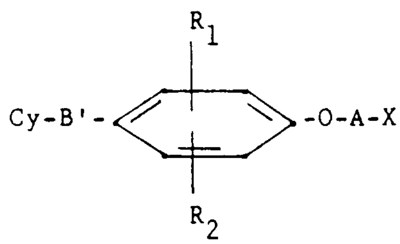

(2)

dans laquelle B' représente un groupement -S- ou $-SO_2-$, Cy, $R_1$ et $R_2$ ont la même signification que dans la Revendication 1, A a la même signification que précédemment et X représente un atome d'halogène ou un groupement alkylsulfonyloxy ayant de 1 à 4 atomes de carbone ou arylsulfonyloxy ayant de 6 à 10 atomes de carbone, avec une amine de formule générale :

H-Am (3)

dans laquelle Am a la même signification que dans la Revendication 1 pour former le dérivé désiré que l'on fait réagir, si on le désire, avec un acide organique ou inorganique pour former un sel pharmaceutiquement acceptable de ce dérivé.

**9.** Procédé de préparation de dérivés d'aminoalkoxyphényles selon la Revendication 1 dans laquelle A représente un radical alkylène et B représente un groupement -S- ou $-SO_2-$, caractérisé en ce que l'on fait réagir, en présence d'un agent basique, un dérivé hydroxy-4 phényle de formule générale :

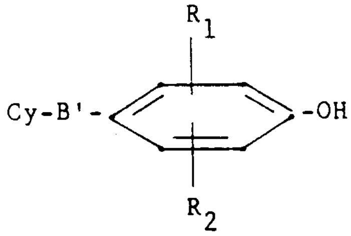

(4)

dans laquelle B' représente un groupement -S- ou $-SO_2-$ et Cy, $R_1$ et $R_2$ ont la même signification que dans la Revendication 1 avec un composé de formule générale :

X-A-Am (52)

dans laquelle X représente un atome d'halogène ou un groupement alkylsulfonyloxy ayant de 1 à 4 atomes de carbone ou arylsulfonyloxy ayant de 6 à 10 atomes de carbone, A représente un radical alkylène et Am a la même valeur que dans la Revendication 1, la réaction ayant lieu au reflux et dans un solvant approprié pour obtenir le dérivé désiré que l'on peut, si on le désire, faire réagir avec un acide organique ou inorganique approprié pour former un sel pharmaceutiquement acceptable.

**10.** Procédé de préparation de dérivés aminoalkoxyphényles selon la Revendication 1 dans laquelle A représente le radical hydroxy-2 propylène éventuellement substitué et B représente un groupement -S- ou $-SO_2-$, caractérisé en ce que l'on traite au reflux un dérivé oxyranylméthoxy de formule générale :

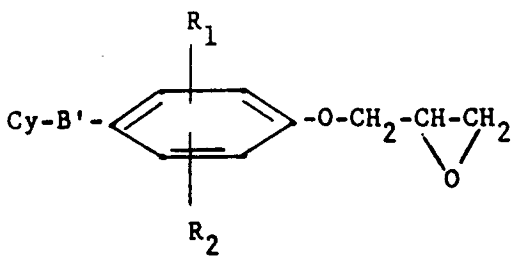

(57)

dans laquelle B' représente un groupement -S- ou $-SO_2-$ et Cy, $R_1$ et $R_2$ ont la même signification que dans la Revendication 1, avec une amine de formule générale :

H-Am (3)

dans laquelle Am a la même signification que dans la Revendication 1 et ce, dans un solvant polaire ou dans un excès de ladite amine pour donner :

- le dérivé désiré, sous forme de base libre, dans lequel A représente un radical hydroxy-2 propylène
- un dérivé aminoalkoxyphényle que l'on peut faire réagir avec un halogénure d'alkyle ayant de 1 à 4 atomes de carbone et en présence d'une base forte, ce qui fournit le dérivé désiré sous forme de base libre, dans lequel A représente un radical hydroxy-2 propylène dans lequel l'hydroxy est substitué par un alkyle ayant de 1 à 4 atomes de carbone,

le dérivé aminoalkoxyphényle ainsi obtenu pouvant, si on le désire, être mis en réaction avec un acide organique ou inorganique pour former un sel pharmaceutiquement acceptable.

**11.** Procédé de préparation de dérivés aminoalkoxyphényles selon la Revendication 1 dans laquelle B représente un groupement -SO-, caractérisé en ce que l'on traite, avec un agent oxydant, un sulfure de formule générale :

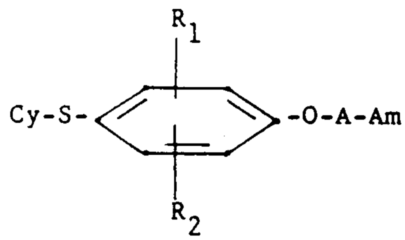

dans laquelle Cy, $R_1$, $R_2$, A et Am ont la même signification que dans la Revendication 1, ce sulfure étant sous forme de base libre ou de sel, pour former le dérivé désiré sous forme de base libre ou de sel que l'on traite avec un agent basique pour fournir le dérivé désiré sous forme de base libre , la base libre ainsi obtenue étant, si on le désire, mise en réaction avec un acide organique ou inorganique pour former un sel pharmaceutiquement acceptable de ce dérivé.

**12.** Procédé selon la Revendication 11 caractérisé en ce que l'agent oxydant est choisi parmi le périodate de sodium, le permanganate de potassium et l'acide chloro-3 perbenzoïque.

**13.** Compositions pharmaceutiques ou vétérinaires contenant, comme principe actif, au moins un dérivé aminoalkoxyphényle selon l'une des Revendications 1 à 6, en association avec un véhicule pharmaceutique ou un excipient approprié.

**14.** Compositions pharmaceutiques ou vétérinaires contenant, comme principe actif, au moins un dérivé aminoalkoxyphényle selon la Revendication 7 en association avec un véhicule pharmaceutique ou un excipient approprié.

**15.** Compositions pharmaceutiques ou vétérinaires selon l'une des Revendications 13 ou 14 pour le traitement de syndromes pathologiques du système cardiovasculaire contenant de 50mg à 500mg de principe actif.

**16.** Utilisation d'au moins un dérivé aminoalkoxyphényle selon l'une quelconque des Revendications 1 à 7 pour la fabrication d'un médicament.

**17.** Utilisation d'au moins un dérivé aminoalkoxyphényle selon l'une quelconque des Revendications 1 à 7 pour la fabrication d'un médicament destiné au traitement de syndromes pathologiques du système cardiovasculaire.

**18.** Utilisation d'au moins un dérivé aminoalkoxyphényle selon l'une des Revendications 1 à 7 pour la fabrication d'un médicament destiné au traitement d'affections pathologiques oculaires.

**19.** Utilisation d'au moins un dérivé aminoalkoxyphényle selon une des Revendications 1 à 7 pour la fabrication d'un médicament destiné à potentialiser les agents anticancéreux.

**Revendications pour l'Etat contractant suivant : ES**

**1.** Procédé de préparation de dérivés aminoalkoxyphényle correspondant à la formule générale :

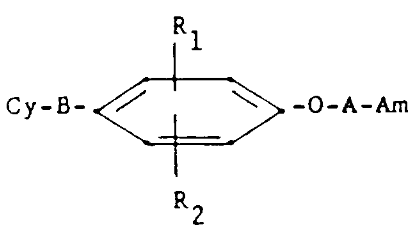

(1)

ainsi que leurs sels pharmaceutiquement acceptables dans laquelle :

B représente un groupement -S-, -SO- ou $-SO_2-$,

$R_1$ et $R_2$, qui sont identiques ou différents, représentent chacun l'hydrogène, le radical méthyle ou éthyle ou un halogène tel que chlore, brome ou iode,

A représente un radical alkylène, linéaire ou ramifié, ayant de 2 à 5 atomes de carbone ou le radical hydroxy-2 propylène dans lequel l'hydroxy est éventuellement substitué par un radical alkyle ayant jusqu'à quatre atomes de carbone,

Am représente un groupement :

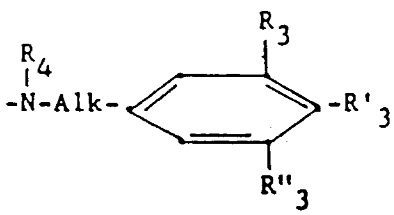

ou

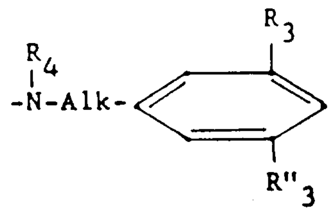

dans lequel : $R_3$, $R'_3$ et $R''_3$, qui sont identiques ou différents, représentent chacun un atome d'halogène tel que chlore ou brome, un groupement alkyle ayant jusqu'à quatre atomes de carbone ou un groupement alkoxy ayant jusqu'à quatre atomes de carbone, ou un groupement benzyloxy,

$R_4$ représente l'hydrogène ou un radical alkyle ayant jusqu'à huit atomes de carbone,

Alk représente une liaison simple ou un radical alkylène, linéaire ou ramifié ayant de 1 à 5 atomes de carbone,

Cy représente un groupement de formule

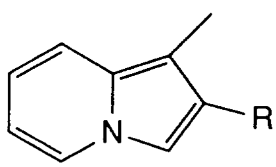

ou

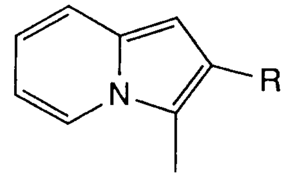

dans lequel

R représente l'hydrogène, un radical alkyle ayant jusqu'à huit atomes de carbone, un radical cycloalkyle en ($C_3$-$C_6$), un radical benzyle ou un radical phényle éventuellement substitué par un ou plusieurs substituants, qui peuvent être identiques ou différents, sélectionnés parmi des atomes d'halogène ou parmi des groupements alkyles ayant jusqu'à quatre atomes de carbone, alkoxy ayant jusqu'à quatre atomes de carbone ou nitro.

caracterisé en ce que

A/ Quand A représente un radical alkylène et B représente un groupement -S- ou $-SO_2-$,

- soit l'on condense, en présence d'un accepteur d' acide, dans un solvant polaire ou non polaire et à une température comprise entre la température ambiante et la température de reflux, un dérivé alcoxy-4 phényle de formule générale :

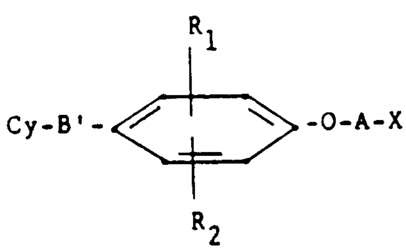

(2)

dans laquelle B' représente un groupement -S- ou $-SO_2-$, Cy, $R_1$ et $R_2$ ont la même signification que précédemment, A a la même signification que précédemment et X représente un atome d'halogène ou un groupement alkylsulfonyloxy ayant de 1 à 4 atomes de carbone ou arylsulfonyloxy ayant de 6 à 10 atomes de carbone, avec une amine de formule générale :

H-Am (3)

dans laquelle Am a la même signification que précédemment, pour former le dérivé désiré sous forme de base libre,

- soit l'on fait réagir, en présence d'un agent basique, un dérivé hydroxy-4 phényle de formule générale :

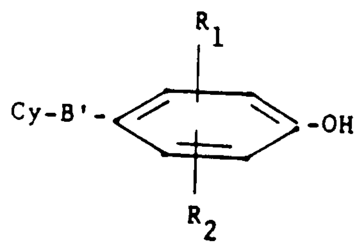

(4)

dans laquelle B' représente un groupement -S- ou $-SO_2-$ et Cy, $R_1$ et $R_2$ ont la même signification que précédemment avec un composé de formule générale :

X-A-Am (52)

dans laquelle X représente un atome d' halogène ou un groupement alcylsulfonyloxy ayant de 1 à 4 atomes de carbone ou arylsulfonyloxy ayant de 6 à 10 atomes de carbone, A représente un radical alkylène et Am a la même valeur que précédemment, la réaction ayant lieu au reflux et dans un solvant approprié pour obtenir le dérivé désiré sous forme de base libre ;

B/ Quand A représente le radical hydroxy-2 propylène éventuellement substitué et B représente un groupent -S- ou $-SO_2-$, l'on traite au reflux un dérivé oxyranylméthoxy de formule générale :

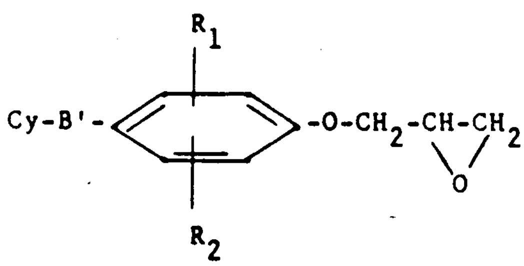

(57)

dans laquelle B' représente un groupement -S- ou $-SO_2-$ et Cy, $R_1$ et $R_2$ ont la même signification que précédemment, avec une amine de formule générale :

H-Am (3)

dans laquelle Am a la même signification que précédemment et ce, dans un solvant polaire ou dans un excès de ladite amine pour donner :

- le dérivé désiré, sous forme de base libre, dans lequel A représente un radical hydroxy-2 propylène.
- un dérivé aminoalkoxyphényle que l'on peut faire réagir avec un halogénure d'alkyle ayant de 1 à 4 atomes de carbone et en présence d'une base forte, ce qui fournit le dérivé désiré sous forme de base libre, dans lequel A représente un radical hydroxy-2 propylène dans lequel l 'hydroxy est substitué par un alkyle ayant de 1 à 4 atomes de carbone, et;

C/ Quand B représente un groupement -SO-, l'on traite, avec un agent oxydant, un sulfure de formule générale :

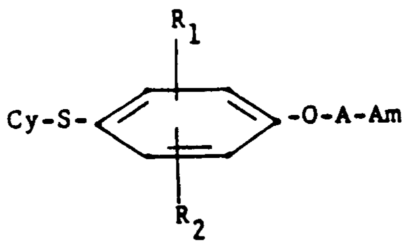

dans lequelle Cy, $R_1$, $R_2$, A et Am ont la même signification que précédemment, ce sulfure étant sous forme de base libre ou de sel, pour former le dérivé désiré sous forme de base libre ou de sel que l'on traite avec un agent basique pour fournir le dérivé désiré sous forme de base libre;
la base libre obtenue étant, si on le désire, mise en réaction avec un acide organique ou inorganique pour former un sel pharmaceutiquement acceptable du dérivé de formule (1).

**2.** Procédé selon la revendication 1 caractérisé en ce que l'agent oxydant est choisi parmi le périodate de sodium, le permanganate de potassium et l'acide chloro-3 perbenzoique.

**3.** Procédé selon la revendication 1, caractérisé en ce que l'on prépare des dérivés aminoalkoxyphényle de formule (1) dans laquelle B représente un groupement $-SO_2-$.

**4.** Procédé selon la revendication 1, caractérisé en ce que l'on prépare des dérivés aminoalkoxyphényle de formule (1) dans laquelle $R_1$ et $R_2$ représentent chacun l'hydrogène.

**5.** Procédé selon la revendication 1, caractérisé en ce que l'on prépare des dérivés aminoalkoxyphényle de formule (1) dans laquelle $R_3$, $R'_3$ et $R''_3$ représentent méthoxy.

**6.** Procédé selon la revendication 1, caractérisé en ce que l'on prépare des dérivés aminoalkoxyphényle de formule (1) dans laquelle R représente le groupement isopropyle ou cyclopropyle.

**7.** Procédé selon la revendication 1, caractérisé en ce que l'on prépare des dérivés aminoalkoxyphényle de formule (1) dans laquelle le sel pharmaceutiquement acceptable est l'oxalate, le chlorhydrate ou le méthanesulfonate.

**8.** Procédé selon la revendication 1, caractérisé en ce que l'on prépare des dérivés aminoalkoxyphényle de formule (1) choisis parmi la
[{[ N-méthyl N-(triméthoxy-3,4,5 β-phénéthyl)amino -3 propyl} oxy-4 benzènesulfonyl] -1 isopropyl-2 indolizine.
la [{[N-méthyl N-(diméthoxy-3-4 méthyl-5 β-phénéthyl)amino] -3 propyl} oxy-4 benzènesulfonyl]-1 isopropyl-2 indolizine,
la [{[N-(diméthoxy-3,5 β-phénéthylamino]-3 propyl} oxy-4 benzènesulfonyl -1 isopropyl-2 indolizine,
et leurs sels pharmaceutiquement acceptables.

**9.** Procédé de préparation de compositions pharmaceutiques ou vétérinaires caractérisé en ce que l'on ajoute au moins un dérivé aminoalkoxyphényle obtenu selon l'une quelconque des revendications précédentes à un véhicule pharmaceutique ou un excipient approprié.

**10.** Procédé de préparation de compositions pharmaceutiques selon la revendication 9 pour le traitement de syndromes pathologiques du système cardio vasculaire, contenant de 50mg à 500mg de principe actif.

**11.** Procédé de préparation de compositions pharmaceutiques selon la revendication 9 pour le traitement d'affections pathologiques oculaires.

**12.** Procédé de préparation de compositions pharmaceutiques selon la revendication 9 pour la potentialisation des agents anticancéreux.

**Revendications pour l'Etat contractant suivant : GR**

**1.** Dérivés aminoalkoxyphényle correspondant à la formule générale :

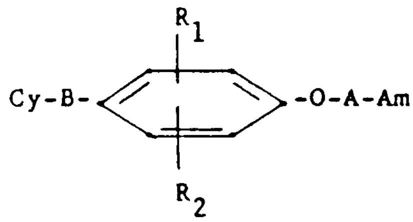

(1)

ainsi que leurs sels pharmaceutiquement acceptables dans laquelle :

B représente un groupement -S-, -SO- ou $-SO_2-$,

$R_1$ et $R_2$, qui sont identiques ou différents, représentent chacun l'hydrogène, le radical méthyle ou éthyle ou un halogène tel que chlore, brome ou iode,

A représente un radical alkylène, linéaire ou ramifié, ayant de 2 à 5 atomes de carbone ou le radical hydroxy-2 propylène dans lequel l'hydroxy est éventuellement substitué par un radical alkyle ayant jusqu'à quatre atomes de carbone,

Am représente un groupement :

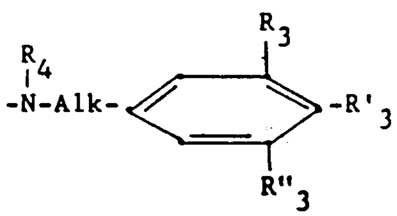

ou

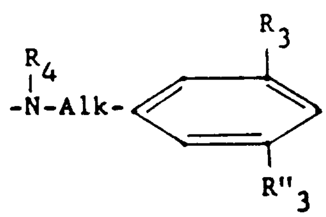

dans lequel : $R_3$, $R'_3$ et $R''_3$, qui sont identiques ou différents, représentent chacun un atome d'halogène tel que chlore ou brome, un groupement alkyle ayant jusqu'à quatre atomes de carbone ou un groupement alkoxy ayant jusqu'à quatre atomes de carbone, ou un groupement benzyloxy,

$R_4$ représente l'hydrogène ou un radical alkyle ayant jusqu'à huit atomes de carbone,

Alk représente une liaison simple ou un radical alkylène, linéaire ou ramifié ayant de 1 à 5 atomes de carbone,

Cy représente un groupement de formule

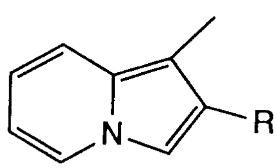

ou

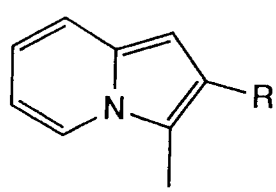

dans lequel

R représente l'hydrogène, un radical alkyle ayant jusqu'à huit atomes de carbone, un radical cycloalkyle en ($C_3$-$C_6$), un radical benzyle ou un radical phényle éventuellement substitué par un ou plusieurs substituants, qui peuvent être identiques ou différents, sélectionnés parmi des atomes d'halogène ou

parmi des groupements alkyles ayant jusqu'à quatre atomes de carbone, alkoxy ayant jusqu'à quatre atomes de carbone ou nitro.

2. Dérivés aminoalkoxyphényle selon la Revendication 1 dans laquelle B représente un groupement $-SO_2-$.

3. Dérivés aminoalkoxyphényles selon la Revendication 1 dans laquelle $R_1$ et $R_2$ représentent chacun l'hydrogène.

4. Dérivés aminoalkoxyphényle selon la Revendication 1 dans laquelle $R_3$, $R'_3$ et $R''_3$ représentent méthoxy.

5. Dérivés aminoalkoxyphényle selon la Revendication 1 dans laquelle R représente le groupement isopropyle ou cyclopropyle.

6. Dérivés aminoalkoxyphényle selon la Revendication 1 dans laquelle le sel pharmaceutiquement acceptable est l'oxalate le chlorhydrate ou le méthanesulfonate.

7. Dérivés d'aminoalkylkoxyphényle selon la revendication 1, choisis parmi :
la [{[N-méthyl N-(triméthoxy-3,4,5 β-phénéthyl)amino]-3propyl} oxy-4 benzènesulfonyl]-1 isopropyl-2 indolizine,
la [{[N-méthyl N-(diméthoxy-3,4 méthyl-5 β-phénéthyl)amino]-3 propyl} oxy-4 benzènesulfonyl]-1 isopropyl-2 indolizine,
la [{[N-diméthoxy-3,5 β-phénéthyl)amino]-3 propyl} oxy-4 benzènesulfonyl}-1 isopropyl-2 indolizine et leurs sels pharmaceutiquement acceptables.

8. Procédé de préparation de dérivés d'aminoalkoxyphényle selon la Revendication 1 dans laquelle A représente un radical alkylène et B représente un groupement -S- ou $-SO_2-$, caractérisé en ce que l'on condense, en présence d'un accepteur d'acide, dans un solvant polaire ou non polaire et à une température comprise entre la température ambiante et la température de reflux, un dérivé alcoxy-4 phényle de formule générale :

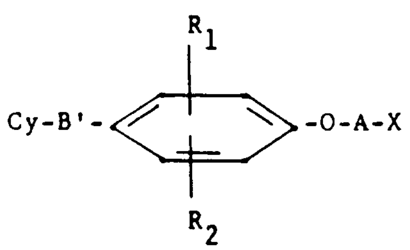

(2)

dans laquelle B' représente un groupement -S- ou $-SO_2-$, Cy, $R_1$ et $R_2$ ont la même signification que dans la Revendication 1, A a la même signification que précédemment et X représente un atome d'halogène ou un groupement alkylsulfonyloxy ayant de 1 à 4 atomes de carbone ou arylsulfonyloxy ayant de 6 à 10 atomes de carbone, avec une amine de formule générale :

H-Am (3)

dans laquelle Am a la même signification que dans la Revendication 1 pour former le dérivé désiré que l'on fait réagir, si on le désire, avec un acide organique ou inorganique pour former un sel pharmaceutiquement acceptable de ce dérivé.

9. Procédé de préparation de dérivés d'aminoalkoxyphényle selon la Revendication 1 dans laquelle A représente un radical alkylène et B représente un groupement -S- ou $-SO_2-$, caractérisé en ce que l'on fait réagir, en présence d'un agent basique, un dérivé hydroxy-4 phényle de formule générale :

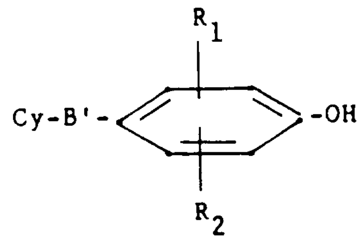

(4)

dans laquelle B' représente un groupement -S- ou $-SO_2-$ et Cy, $R_1$ et $R_2$ ont la même signification que dans la Revendication 1 avec un composé de formule générale :

X-A-Am (52)

dans laquelle X représente un atome d'halogène ou un groupement alkylsulfonyloxy ayant de 1 à 4 atomes de carbone ou arylsulfonyloxy ayant de 6 à 10 atomes de carbone, A représente un radical alkylène et Am a la même valeur que dans la Revendication 1, la réaction ayant lieu au reflux et dans un solvant approprié pour obtenir le dérivé désiré que l'on peut, si on le désire, faire réagir avec un acide organique ou inorganique approprié pour former un sel pharmaceutiquement acceptable.

**10.** Procédé de préparation de dérivés aminoalkoxyphényle selon la Revendication 1 dans laquelle A représente le radical hydroxy-2 propylène éventuellement substitué et B représente un groupement -S- ou $-SO_2-$, caractérisé en ce que l'on traite au reflux un dérivé oxyranylméthoxy de formule générale :

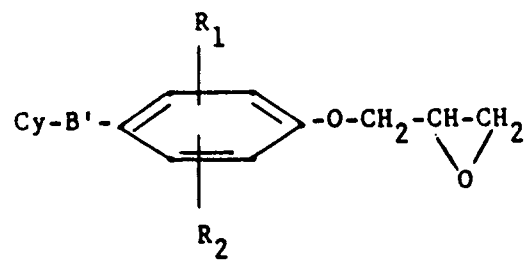

(57)

dans laquelle B' représente un groupement -S- ou $-SO_2-$ et Cy, $R_1$ et $R_2$ ont la même signification que dans la Revendication 1, avec une amine de formule générale :

H-Am (3)

dans laquelle Am a la même signification que dans la Revendication 1 et ce, dans un solvant polaire ou dans un excès de ladite amine pour donner :

- le dérivé désiré, sous forme de base libre, dans lequel A représente un radical hydroxy-2 propylène
- un dérivé aminoalkoxyphényle que l'on peut faire réagir avec un halogénure d'alkyle ayant de 1 à 4 atomes de carbone et en présence d'une base forte, ce qui fournit le dérivé désiré sous forme de base libre, dans lequel A représente un radical hydroxy-2 propylène dans lequel l'hydroxy est substitué par un alkyle ayant de 1 à 4 atomes de carbone,

le dérivé aminoalkoxyphényle ainsi obtenu pouvant, si on le désire, être mis en réaction avec un acide organique ou inorganique pour former un sel pharmaceutiquement acceptable.

**11.** Procédé de préparation de dérivés aminoalkoxyphényle selon la Revendication 1 dans laquelle B représente un groupement -SO-, caractérisé en ce que l'on traite, avec un agent oxydant, un sulfure de formule générale :

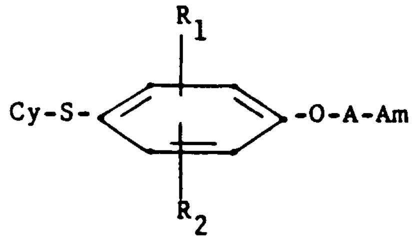

dans laquelle Cy, $R_1$, $R_2$, A et Am ont la même signification que dans la Revendication 1, ce sulfure étant sous forme de base libre ou de sel, pour former le dérivé désiré sous forme de base libre ou de sel que l'on traite avec un agent basique pour fournir le dérivé désiré sous forme de base libre, la base libre ainsi obtenue étant, si on le désire, mise en réaction avec un acide organique ou inorganique pour former un sel pharmaceutiquement acceptable de ce dérivé.

**12.** Procéde selon la Revendication 11 caractérisé en ce que l'agent oxydant est choisi parmi le périodate de sodium, le permanganate de potassium et l'acide chloro-3 perbenzoique.

**13.** Procédé de préparation de compositions pharmaceutiques ou vétérinaires caractérisé en ce que l'on additionne, au moins un dérivé aminoalkoxyphényle selon l'une des Revendications 1 à 6, avec un véhicule pharmaceutique ou un excipient approprié.

**14.** Procédé de préparation de compositions pharmaceutiques ou vétérinaires caractérisé en ce que l'on additionne au moins un dérivé aminoalkoxyphényle selon la Revendication 7 avec un véhicule pharmaceutique ou un excipient approprié.

**15.** Procédé de préparation de compositions pharmaceutiques ou vétérinaires selon l'une des Revendications 13 ou 14 pour le traitement de syndromes pathologiques du système cardiovasculaire contenant de 50mg à 500mg de principe actif.

**16.** Procédé de préparation de compositions pharmaceutiques ou vétérinaires selon l'une des Revendications 13 ou 14 destinées au traitement d'affections pathologiques oculaires.

**17.** Procédé de préparation de compositions pharmaceutiques ou vétérinaires selon l'une des Revendications 13 ou 14 destinées à potentialiser les agents anticancéreux.

**Claims**

**Claims for the following Contracting States : AT, BE, CH, DE, DK, FR, GB, IT, LI, LU, NL, SE**

**1.** Aminoalkoxyphenyl derivatives corresponding to the general formula:

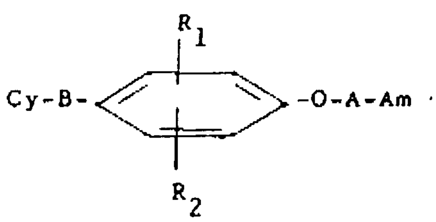

(I)

and the pharmaceutically acceptable salts thereof wherein:
B denotes a -S-, -SO- or $-SO_2-$ group,
$R_1$ and $R_2$, which are identical or different, each denote hydrogen, a methyl or ethyl radical or a halogen such as chlorine, bromine or iodine,
A denotes a straight chained or branched $C_{2-5}$ alkylene radical or a 2-hydroxy propylene radical, wherein the hydroxy is optionally substituted by an alkyl radical having up to 4 carbon atoms,
Am denotes a group:

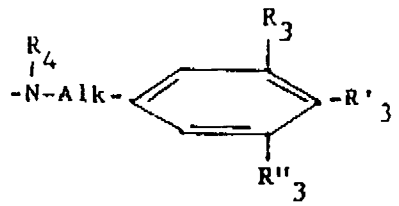

or

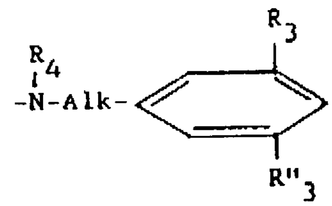

wherein:

$R_3$, $R'_3$ and $R''_3$ which are identical or different, each denote a halogen atom such as chlorine or bromine, an alkyl group having up to four carbon atoms or an alkoxy group having up to four carbon atoms, or a benzyloxy group,

$R_4$ denotes hydrogen or an alkyl radical having up to eight carbon atoms,

Alk represents a single bond or a straight chained or branched $C_{1-5}$ alkylene radical,

Cy represents a group of formula:

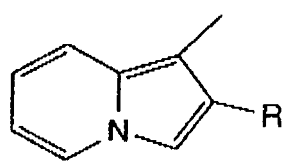

or

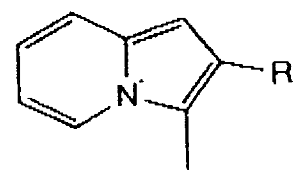

wherein R denotes hydrogen, an alkyl radical having up to eight carbon atoms, a $C_{3-6}$ cycloalkyl radical, a benzyl radical or a phenyl radical optionally substituted by one or more substituents, which may be identical or different, selected from among the halogen atoms or from alkyl groups having up to four carbon atoms, alkoxy groups having up to four carbon atoms or nitro groups.

**2.** Aminoalkoxyphenyl derivatives according to claim 1, wherein B represents a group $-SO_2-$.

**3.** Aminoalkoxyphenyl derivatives according to claim 1, wherein $R_1$ and $R_2$ each denote hydrogen.

**4.** Aminoalkoxyphenyl derivatives according to claim 1, wherein $R_3$, $R'_3$ and $R''_3$ represent methoxy.

**5.** Aminoalkoxyphenyl derivatives according to claim 1, wherein R denotes the isopropyl or a cyclopropyl group.

**6.** Aminoalkoxyphenyl derivatives according to claim 1, wherein the pharmaceutically acceptable salt is the oxylate, hydrochloride or methane sulphonate.

**7.** Aminoalkoxyphenyl derivatives according to claim 1, selected from among:
1-[4-(3-[N-methyl-N-(3,4,5-trimethoxy-β-phenethyl)amino]-propyloxy)-benzenesulphonyl]-2-isopropylindolizine,
1-[4-(3-[N-methyl-N-(3,4-dimethoxy-5-methyl-β-phenethyl)-amino]-propyloxy)-benzenesulphonyl]-2-isopropylindolizine,
1-[4-(3-[N-3,5-dimethoxy-β-phenethyl)amino]-propyloxy)-benzenesulphonyl]-2-isopropylindolizine
and the pharmaceutically acceptable salts thereof.

**8.** Process for preparing aminoalkoxyphenyl derivatives according to claim 1, wherein A denotes an alkaline radical and B denotes an -S- or $-SO_2-$ group,
characterised in that a 4-alkoxyphenyl derivative of general formula:

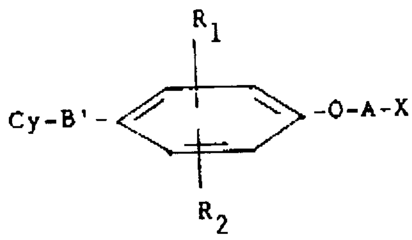

(2)

wherein B' denotes an -S- or $-SO_2-$ group, Cy, $R_1$ and $R_2$ are defined as in claim 1, A is defined as hereinbefore and X denotes a halogen atom or a $C_{1-4}$ alkyl sulphonyloxy group or a $C_{6-10}$ arylsulphonyloxy group, is condensed, in the presence of an acid acceptor, in a polar or non-polar solvent and at a temperature between ambient temperature and reflux temperature, with an amine of general formula:

H-Am (3)

wherein Am is defined as in claim 1 to form the desired derivative which is reacted, if desired, with an organic or inorganic acid in order to form a pharmaceutically acceptable salt of this derivative.

**9.** Process for preparing aminoalkoxyphenyl derivatives according to claim 1, wherein A represents an alkylene radical and B represents an -S- or $-SO_2-$ group,
characterised in that a 4-hydroxyphenyl derivative of general formula:

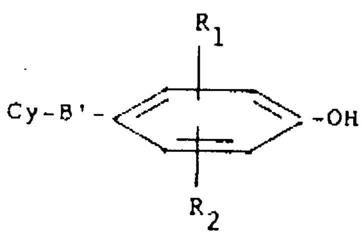

(4)

wherein B' represents an -S- or $-SO_2-$ group and Cy, $R_1$ and $R_2$ are defined as in claim 1, is reacted, in the presence of a basic agent, with a compound of general formula:

X-A-Am (52)

wherein X denotes a halogen atom or a $C_{1-4}$ alkylsulphonyloxy group or a $C_{6-10}$ aryl sulphonyloxy group, A denotes an alkylene radical and Am has the same value as in claim 1, the reaction being carried out by refluxing, in an appropriate solvent, in order to obtain the desired derivative which may, if desired, be reacted with an appropriate organic or inorganic acid to form a pharmaceutically acceptable salt.

**10.** Process for preparing aminoalkoxyphenyl derivatives according to claim 1, wherein A represents an optionally substituted 2-hydroxypropylene radical and B denotes an -S- or $-SO_2-$ group, characterized in that an oxyranylmethoxy derivative of general formula:

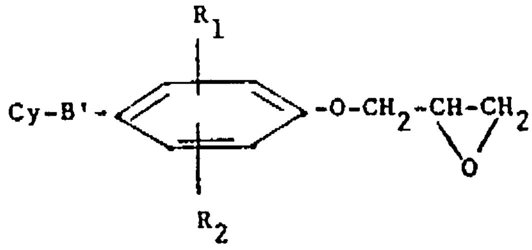

(57)

wherein B' denotes an -S- or $-SO_2-$ group and Cy, $R_1$ and $R_2$ are defined as in claim 1, is refluxed with an amine of general formula:

H-Am (3)

wherein Am is defined as in claim 1, in a polar solvent or in an excess of the amine in order to give:

- the desired derivative, in the form of a free base, wherein A denotes a 2-hydroxypropylene radical,
- an aminoalkoxylphenyl derivative which may be reacted with a $C_{1-4}$ alkyl halide in the presence of a strong base, yielding the desired derivatives in the form of a free base, wherein A denotes a 2-hydroxypropylene radical wherein the hydroxy is substituted by a $C_{1-4}$ alkyl,

the aminoalkoxyphenyl derivative thus obtained being capable of being reacted, if desired, with an organic or inorganic acid in order to form a pharmaceutically acceptable salt.

**11.** Process for preparing aminoalkoxylphenyl derivatives according to claim 1, wherein B denotes an SO-group, characterised in that a sulphide of general formula:

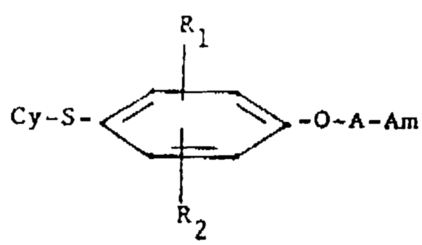

wherein Cy, $R_1$, $R_2$, A and Am are defined as in claim 1, this sulphide being in the form of a free base or a salt, is treated with an oxidising agent, to form the desired derivative in the form of a free base or a salt which is treated with a basic agent to obtain the desired derivative in the form of a free base, the free base thus obtained being reacted, if desired, with an organic or inorganic acid to form a pharmaceutically acceptable salt of this derivative.

**12.** Process according to claim 11, characterised in that the oxidising agent is selected from sodium periodate, potassium permanganate and 3-chloroperbenzoic acid.

**13.** Pharmaceutical or veterinary compositions containing, as active principle, at least one aminoalkoxyphenyl derivative according to one of claims 1 to 6, combined with a pharmaceutical carrier or a suitable excipient.

**14.** Pharmaceutical or veterinary compositions containing, as active principle, at least one aminoalkoxyphenyl derivative according to claim 7 combined with a pharmaceutical carrier or a suitable excipient.

**15.** Pharmaceutical or veterinary compositions according to one of claims 13 and 14 for the treatment of pathological syndromes of the cardiovascular system, containing 50 to 500 mg of active principle.

**16.** Use of at least one aminylalkoxypenyl derivative according to any one of claims 1 to 7 for the production of a drug.

**17.** Use of at least one aminoalkoxyphenyl derivative according to any one of claims 1 to 7 for the production of a drug intended for the treatment of pathological syndromes of the cardiovascular system.

**18.** Use of at least one aminoalkoxyphenyl derivative according to one of claims 1 to 7 for the production of a drug intended for the treatment of pathological ocular complaints.

**19.** Use of at least one aminoalkoxyphenyl derivative according to one of claims 1 to 7, for the production of a drug intended to potentiate anti-cancer agents.

**Claims for the following Contracting State : ES**

**1.** Process for preparing aminoalkoxyphenyl derivatives corresponding to the general formula:

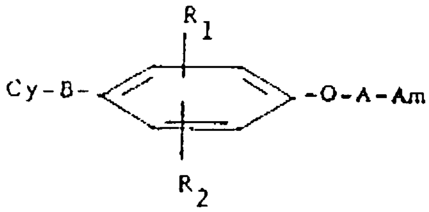

(I)

and the pharmaceutically acceptable salts thereof wherein:
B denotes an -S-, -SO- or -$SO_2$- group,
$R_1$ and $R_2$, which are identical or different, each denote hydrogen, a methyl or ethyl radical or a halogen such as chlorine, bromine or iodine,
A denotes a straight chained or branched $C_{2-5}$ alkylene radical or a 2-hydroxy propylene radical, wherein the hydroxy is optionally substituted by an alkyl radical having up to 4 carbon atoms,
Am denotes a group:

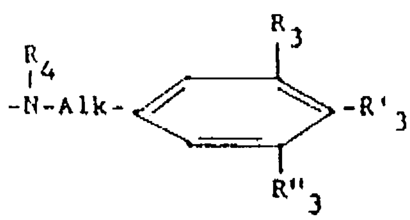

or

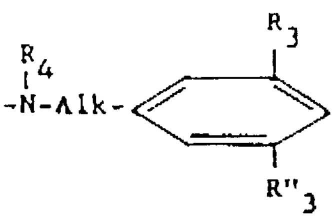

wherein:
$R_3$, $R'_3$ and $R''_3$ which are identical or different, each denote a halogen atom such as chlorine or bromine, an alkyl group having up to four carbon atoms or an alkoxy group having up to four carbon atoms, or a benzyloxy group,
$R_4$ denotes hydrogen or an alkyl radical having up to eight carbon atoms,
Alk represents a single bond or a straight chained or branched $C_{1-5}$ alkylene radical,
Cy represents a group of formula:

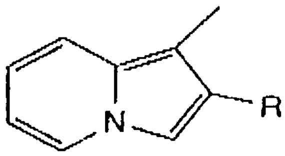

or

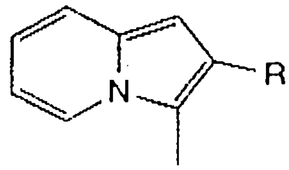

wherein R denotes hydrogen, an alkyl radical having up to eight carbon atoms, a $C_{3-6}$ cycloalkyl radical, a benzyl radical or a phenyl radical optionally substituted by one or more substituents, which may be identical or different, selected from among the halogen atoms or from alkyl groups having up to four carbon atoms, alkoxy groups having up to four carbon atoms or nitro groups, characterised in that

A) when A denotes an alkyline radical and B denotes an -S- or $-SO_2-$ group,
either a 4-alkoxyphenyl derivative of general formula

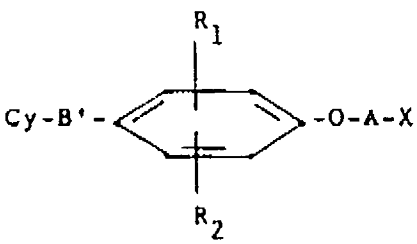

(2)

wherein B' represents an -S- or $-SO_2-$ group, Cy, $R_1$ and $R_2$ are defined as hereinbefore, A is defined as hereinbefore and X denotes a halogen atom or a $C_{1-4}$ alkyl sulphonyloxy group or a $C_{6-10}$ arylsulphonyloxy group, is condensed, in the presence of an acid acceptor in a polar or non-polar solvent and at a temperature between ambient temperature and the reflux temperature, with an amine of general formula:

H-Am (3)

wherein Am is as hereinbefore defined, in order to form the desired derivative in the form of a free base, or a 4-hydroxyphenyl derivative of general formula:

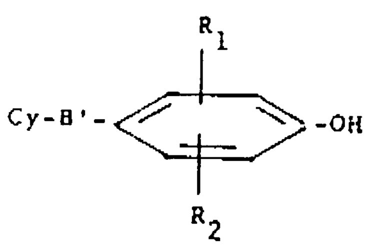

(4)

wherein B' represents an -S- or $-SO_2-$ group and Cy, $R_1$ and $R_2$ are as hereinbefore defined, is reacted, in the presence of a basic agent, with a compound of general formula

X-A-Am (52)

wherein X denotes a halogen atom or a $C_{1-4}$ alkylsulphonyloxy group or a $C_{6-10}$ arylsulphonyloxy group, A denotes an alkaline radical and Am is as hereinbefore defined, the reaction being carried out by refluxing in an appropriate solvent in order to obtain the desired derivative in the form of a free base;

B) when A represents the optionally substituted 2-hydroxypropylene radical and B represents an -S- or $-SO_2$-group, an oxyranylmethoxy derivative of general formula

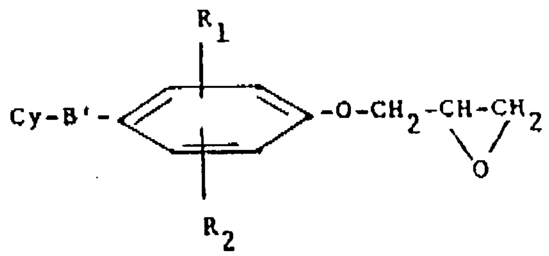

(57)

wherein B' represents an -S- or $-SO_2-$ group and Cy, $R_1$ and $R_2$ are as hereinbefore defined, is refluxed with an amine of general formula:

H-Am (3)

wherein Am is as hereinbefore defined, in a polar solvent or in an excess of the said amine to give:
- the desired derivative, in the form of a free base, wherein A represents a 2-hydroxypropylene radical,
- an aminoalkoxyphenyl derivative which can be reacted with an alkyl halide having 1 to 4 carbon atoms in the presence of a strong base, to yield the desired derivative in the form of a free base, wherein A denotes a 2-hydroxypropylene radical in which the hydroxy is substituted by a $C_{1-4}$ alkyl, and:

C) when B represents an -SO- group, a sulphide of general formula:

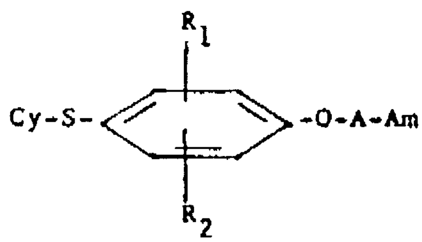

wherein Cy, $R_1$, $R_2$, A and Am are as hereinbefore defined, this sulphide being in the form a free base or a salt, is treated with an oxidising agent in order to form the desired derivative in the form of a free base or salt which is treated with a basic agent to yield the desired derivative in the form of a free base; the free base obtained being reactive, if desired, with an organic or inorganic acid to form a pharmaceutically acceptable salt of the derivative of formula 1.

**2.** Process according to claim 1, characterised in that the oxidising agent is selected from sodium periodate, potassium permanganate and 3-chloroperbenzoic acid.

**3.** Process according to claim 1, characterized in that aminoalkoxyphenyl derivatives of formula 1 are prepared wherein B denotes an $-SO_2-$ group.

**4.** Process according to claim 1, characterised in that aminoalkoxyphenyl derivatives of formula 1 are prepared wherein $R_1$ and $R_2$ each represent hydrogen.

**5.** Process according to claim 1, characterised in that aminoalkoxyphenyl derivatives of formula 1 are prepared wherein $R_3$' $R'_3$ and $R''_3$ represent methoxy.

**6.** Process according to claim 1, characterised in that aminoalkoxyphenyl derivatives of formula 1 are prepared wherein R represents the isopropyl or a cyclopropyl group.

**7.** Process according to claim 1, characterized in that aminoalkoxyphenyl derivatives of formula 1 are prepared wherein the pharmaceutically acceptable salt is the oxylate, hydrochloride or methane sulphonate.

**8.** Process according to claim 1, characterized in that aminoalkoxyphenyl derivatives of formula 1 are prepared which are selected from:
Aminoalkoxyphenyl derivatives according to claim 1, selected from among:
1-[4-(3-[N-methyl-N-(3,4,5-trimethoxy-β-phenethyl)amino]-propyloxy)-benzenesulphonyl]-2-isopropylindolizine,
1-[4-(3-[N-methyl-N-(3,4-dimethoxy-5-methyl-β-phenethyl)-amino]-propyloxy)-benzenesulphonyl]-2-isopropylindolizine,
1-[4-(3-[N-3,5-dimethoxy-β-phenethyl)amino]-propyloxy)-benzenesulphonyl]-2-isopropylindolizine
and the pharmaceutically acceptable salts thereof.

**9.** Process for preparing pharmaceutical or veterinary compositions, characterised in that at least one aminoalkoxyphenyl derivative obtained according to any one of the preceding claims is added to a pharmaceutical carrier or a suitable excipient.

**10.** Process for preparing pharmaceutical compositions according to claim 9 for treating pathological syndromes of the cardiovascular system, containing from 50 to 500mg of active principle.

**11.** Process for preparing pharmaceutical compositions according to claim 9 for the treatment of pathological ocular complaints.

**12.** Process for preparing pharmaceutical compositions according to claim 9 for potentiating anti-cancer agents.

**Claims for the following Contracting State : GR**

**1.** Aminoalkoxyphenyl derivatives corresponding to the general formula:

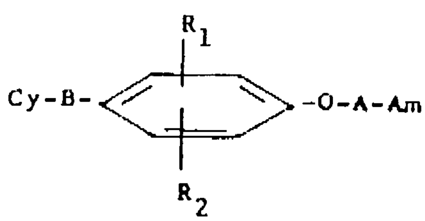

(I)

and the pharmaceutically acceptable salts thereof wherein:
B denotes an -S-, -SO- or -$SO_2$- group,
$R_1$ and $R_2$, which are identical or different, each denote hydrogen, a methyl or ethyl radical or a halogen such as chlorine, bromine or iodine,
A denotes a straight chained or branched $C_{2-5}$ alkylene radical or a 2-hydroxy propylene radical, wherein the hydroxy is optionally substituted by an alkyl radical having up to 4 carbon atoms,
Am denotes a group:

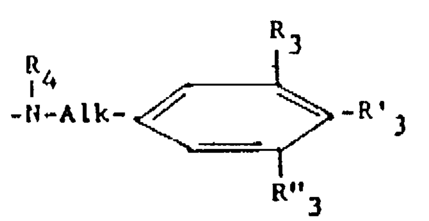

or

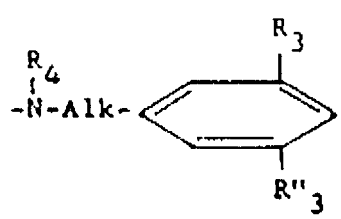

wherein:
$R_3$, $R'_3$ and $R''_3$ which are identical or different, each denote a halogen atom such as chlorine or bromine, an alkyl group having up to four carbon atoms or an alkoxy group having up to four carbon atoms, or a benzyloxy group,
$R_4$ denotes hydrogen or an alkyl radical having up to eight carbon atoms,

Alk represents a single bond or a straight chained or branched $C_{1-5}$ alkylene radical,
Cy represents a group of formula:

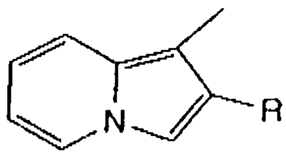

or

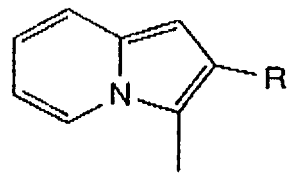

wherein R denotes hydrogen, an alkyl radical having up to eight carbon atoms, a $C_{3-6}$ cycloalkyl radical, a benzyl radical or a phenyl radical optionally substituted by one or more substituents, which may be identical or different, selected from among the halogen atoms or from alkyl groups having up to four carbon atoms, alkoxy groups having up to four carbon atoms or nitro groups.

**2.** Aminoalkoxyphenyl derivatives according to claim 1, wherein B represents a group $-SO_2-$.

**3.** Aminoalkoxyphenyl derivatives according to claim 1, wherein $R_1$ and $R_2$ each denote hydrogen.

**4.** Aminoalkoxyphenyl derivatives according to claim 1, wherein $R_3$, $R'_3$ and $R''_3$ represent methoxy.

**5.** Aminoalkoxyphenyl derivatives according to claim 1, wherein R denotes the isopropyl or a cyclopropyl group.

**6.** Aminoalkoxyphenyl derivatives according to claim 1, wherein the pharmaceutically acceptable salt is the oxylate, hydrochloride or methane sulphonate.

**7.** Aminoalkoxyphenyl derivatives according to claim 1, selected from among:
1-[4-(3-[N-methyl-N-(3,4,5-trimethoxy-β-phenethyl)amino]-propyloxy)-benzenesulphonyl]-2-isopropylindolizine,
1-[4-(3-[N-methyl-N-(3,4-dimethoxy-5-methyl-β-phenethyl)-amino]-propyloxy)-benzenesulphonyl]-2-isopropylindolizine,
1-[4-(3-[N-3,5-dimethoxy-β-phenethyl)amino]-propyloxy)-benzenesulphonyl]-2-isopropylindolizine
and the pharmaceutically acceptable salts thereof.

**8.** Process for preparing aminoalkoxyphenyl derivatives according to claim 1, wherein A denotes an alkaline radical and B denotes an -S- or $-SO_2-$ group,
characterized in that a 4-alkoxyphenyl derivatives of general formula:

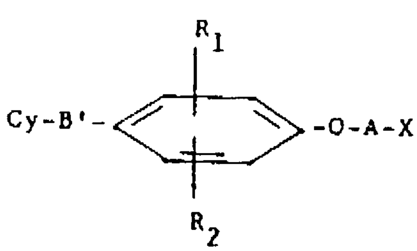

(2)

wherein B' denotes an -S- or $-SO_2-$ group, Cy, $R_1$ and $R_2$ are defined as in claim 1, A is defined as hereinbefore and X denotes a halogen atom or a $C_{1-4}$ alkyl sulphonyloxy group or a $C_{6-10}$ arylsulphonyloxy group, is condensed, in the presence of an acid acceptor, in a polar or non-polar solvent and at a temperature between ambient temperature and reflux temperature, with an amine of general formula:

H-Am (3)

wherein Am is defined as in claim 1 to form the desired derivative which is reacted, if desired, with an organic or inorganic acid in order to form a pharmaceutically acceptable salt of this derivative.

**9.** Process for preparing aminoalkoxyphenyl derivatives according to claim 1, wherein A represents an alkylene radical and B represents an -S- or $-SO_2-$ group,
characterised in that a 4-hydroxyphenyl derivative of general formula:

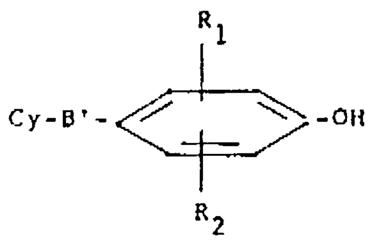

(4)

wherein B' represents an -S- or $-SO_2-$ group and Cy, $R_1$ and $R_2$ are defined as in claim 1, is reacted, in the presence of a basic agent, with a compound of general formula:

X-A-Am (52)

wherein X denotes a halogen atom or a $C_{1-4}$ alkylsulphonyloxy group or a $C_{6-10}$ aryl sulphonyloxy group, A denotes an alkylene radical and Am has the same value as in claim 1, the reaction being carried out by refluxing, in an appropriate solvent, in order to obtain the desired derivative which may, if desired, be reacted with an appropriate organic or inorganic acid to form a pharmaceutically acceptable salt.

**10.** Process for preparing aminoalkoxyphenyl derivatives according to claim 1, wherein A represents an optionally substituted 2-hydroxypropylene radical and B denotes an -S- or $-SO_2-$ group, characterised in that an oxyranylmethoxy derivative of general formula:

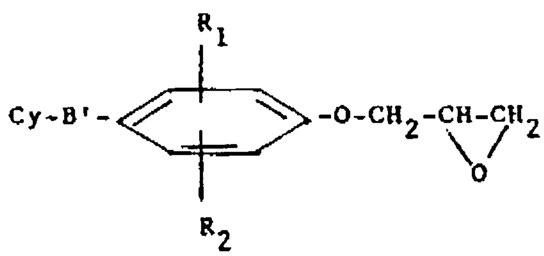

(57)

wherein B' denotes an -S- or $-SO_2-$ group and Cy, R1 and R2 are defined as in claim 1, is refluxed with an amine of general formula:

H-Am (3)

wherein Am is defined as in claim 1, in a polar solvent or in an excess of the amine in order to give:

- the desired derivative, in the form of a free base,
- wherein A denotes a 2-hydroxypropylene radical, an aminoalkoxylphenyl derivative which may be reacted with a $C_{1-4}$ alkyl halide in the presence of a strong base, yielding the desired derivatives in the form of a free base, wherein A denotes a 2-hydroxypropylene radical wherein the hydroxy is substituted by a $C_{1-4}$ alkyl,

the aminoalkoxyphenyl derivative thus obtained being capable of being reacted, if desired, with an organic or inorganic acid in order to form a pharmaceutically acceptable salt.

**11.** Process for preparing aminoalkoxylphenyl derivatives according to claim 1, wherein B denotes an -SO- group, characterised in that a sulphide of general formula:

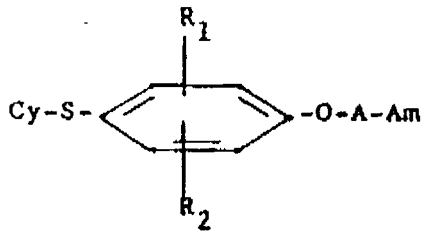

wherein Cy, $R_1$, $R_2$, A and Am are defined as in claim 1, this sulphide being in the form of a free base or a salt, is treated with an oxidising agent, to form the desired derivative in the form of a free base or a salt which is treated with a basic agent to obtain the desired derivative in the form of a free base, the free base thus obtained being reacted, if desired, with an organic or inorganic acid to form a pharmaceutically acceptable salt of this derivative.

**12.** Process according to claim 11, characterised in that the oxidising agent is selected from sodium periodate, potassium permanganate and 3-chloroperbenzoic acid.

**13.** Process for preparing pharmaceutical or veterinary compositions, characterised in that at least one aminoalkoxyophenyl derivative according to one of claims 1-6 is combined with a pharmaceutical carrier or a suitable excipient.

**14.** Process for preparing pharmaceutical or veterinary compositions, characterised in that at least one aminoalkoxyphenyl derivative according to claim 7 is combined with a pharmaceutical carrier or a suitable excipient.

**15.** Process for preparing pharmaceutical or veterinary compositions according to one of claims 13 or 14 for treating pathological syndromes of the cardiovascular system, containing from 50 to 500mg of active principle.

**16.** Process for preparing pharmaceutical or veterinary compositions according to one of claims 13 and 14, intended for treating pathological ocular complaints.

**17.** Process for preparing pharmaceutical or veterinary compositions according to one of claims 13 and 14, intended for potentiating anti-cancer agents.

**Patentansprüche**

**Patentansprüche für folgende Vertragsstaaten : AT, BE, CH, DE, DK, FR, GB, IT, LI, LU, NL, SE**

**1.** Aminoalkoxyphenylderivate der allgemeinen Formel:

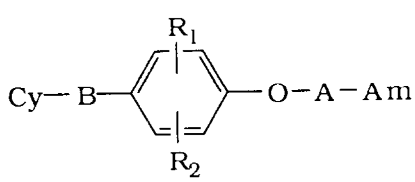

(1)

sowie deren pharmazeutisch annehmbare Salze, worin:

| | |
|---|---|
| B | eine -S-, -SO- oder $-SO_2$-Gruppe, |
| $R_1$ und $R_2$, | die gleichartig oder verschieden sind, jeweils Wasserstoff, eine Methyl- oder Ethylgruppe oder ein Halogenatom, wie ein Chlor-, Brom- oder Iodatom, |
| A | eine geradkettige oder verzweigte Alkylengruppe mit 2 bis 5 Kohlenstoffatomen oder die 2-Hydroxy-propylengruppe, worin die Hydroxylgruppe gegebenenfalls durch eine Alkylgruppe mit bis zu vier Kohlenstoffatomen substituiert ist, |
| Am | eine Gruppe: |

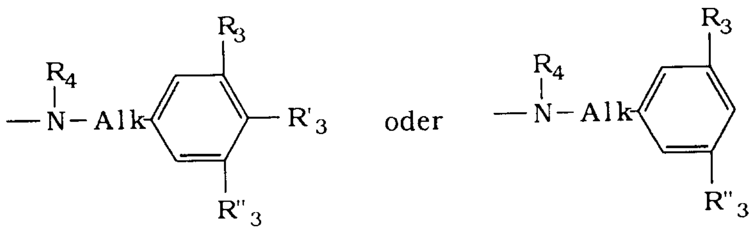

in denen $R_3$, $R'_3$ und $R''_3$, die gleichartig oder verschieden sind, jeweils ein Halogenatom, wie ein Chlor- oder ein Bromatom, eine Alkylgruppe mit bis zu vier Kohlenstoffatomen oder eine Alkoxygruppe mit bis zu vier Kohlenstoffatomen oder eine Benzyloxygruppe,

$R_4$ ein Wasserstoffatom oder eine Alkylgruppe mit bis zu acht Kohlenstoffatomen,

Alk eine Einfachbindung oder eine geradkettige oder verzweigte Alkylengruppe mit 1 bis 5 Kohlenstoffatomen darstellen und

Cy eine Gruppe der Formeln

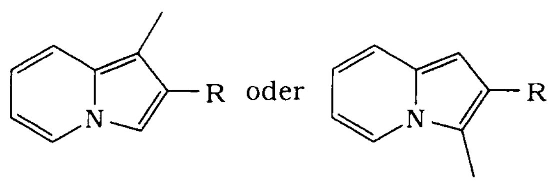

worin

R ein Wasserstoffatom, eine Alkylgruppe mit bis zu acht Kohlenstoffatomen, eine $C_3$-$C_6$-Cycloalkylgruppe, eine Benzylgruppe oder eine Phenylgruppe, die gegebenenfalls durch einen oder mehrere Substituenten substituiert ist, die gleichartig oder verschieden sein können und aus Halogenatomen oder Alkylgruppen mit bis zu vier Kohlenstoffatomen, Alkoxygruppen mit bis zu vier Kohlenstoffatomen und Nitrogruppen ausgewählt sind, substituiert sein können, darstellt, bedeuten.

**2.** Aminoalkoxyphenylderivate nach Anspruch 1, worin B eine -$SO_2$-Gruppe bedeutet.

**3.** Aminoalkoxyphenylderivate nach Anspruch 1, worin $R_1$ und $R_2$ jeweils Wasserstoff bedeuten.

**4.** Aminoalkoxyphenylderivate nach Anspruch 1, worin $R_3$, $R'_3$ und $R''_3$ Methoxygruppen bedeuten.

**5.** Aminoalkoxyphenylderivate nach Anspruch 1, worin R die Isopropyl- oder Cyclopropylgruppe bedeutet.

**6.** Aminoalkoxyphenylderivate nach Anspruch 1, worin das pharmazeutisch annehmbare Salz das Oxalat, das Hydrochlorid oder das Methansulfonat ist.

**7.** Aminoalkoxyphenylderivate nach Anspruch 1 ausgewählt aus:
1-[{3-[N-Methyl-N-(3,4,5-trimethoxy-β-phenethyl}-amino]-propyl}-4-oxy-benzolsulfonyl]-2-isopropyl-indolizin,
1-{[3-[N-Methyl-N-(3,4-dimethoxy-5-methyl-β-phenethyl)-amino]-propyl}-4-oxy-benzolsulfonyl]-2-isopropyl-indolizin,
1-[{3-[N-[3,5-Dimethoxy-β-phenethyl)-amino]-propyl}-4-oxy-benzolsulfonyl]-2-isopropyl-indolizin
und dessen pharmazeutisch annehmbaren Salzen.

**8.** Verfahren zur Herstellung der Aminoalkoxyphenylderivate nach Anspruch 1, worin A eine Alkylengruppe und B eine -S- oder -$SO_2$-Gruppe bedeuten, **dadurch gekennzeichnet**, daß man ein 4-Alkoxyphenylderivat der allgemeinen Formel:

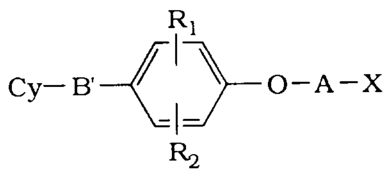

(2)

in der B' eine -S- oder $-SO_2$-Gruppe darstellt, Cy, $R_1$ und $R_2$ die in Anspruch 1 angegebenen Bedeutungen besitzen, A die oben angegebenen Bedeutungen besitzt und X ein Halogenatom oder eine Alkylsulfonyloxygruppe mit 1 bis 4 Kohlenstoffatomen oder eine Arylsulfonyloxygruppe mit 6 bis 10 Kohlenstoffatmen darstellt,
in Gegenwart eines Säureakzeptors in einem polaren oder nichtpolaren Lösungsmittel bei einer Temperatur zwischen Raumtemperatur und der Rückflußtemperatur mit einem Amin der allgemeinen Formel:

H - Am (3)

in der Am die in Anspruch 1 angegebenen Bedeutungen besitzt, kondensiert zur Bildung des gewünschten Derivats, welches man gewünschtenfalls mit einer organischen oder anorganischen Säure zur Bildung eines pharmazeutisch annehmbaren Salzes dieses Derivats umsetzt.

**9.** Verfahren zur Herstellung der Aminoalkoxyphenylderivate nach Anspruch 1, worin A eine Alkylengruppe und B eine -S- oder $-SO_2$-Gruppe bedeuten, **dadurch gekennzeichnet,** daß man ein 4-Hydroxyphenylderivat der allgemeinen Formel:

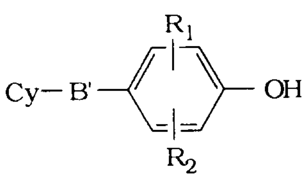

(4)

in der B' eine -S- oder $-SO_2$-Gruppe darstellt und Cy, $R_1$ und $R_2$ die in Anspruch 1 angegebenen Bedeutungen besitzen, in Gegenwart eines basischen Mittels mit einer Verbindung der allgemeinen Formel:

X - A - Am (52)

in der X ein Halogenatom oder eine Alkylsulfonyloxygruppe mit 1 bis 4 Kohlenstoffatomen oder eine Arylsulfonyloxygruppe mit 6 bis 10 Kohlenstoffatomen und A eine Alkylengruppe darstellen und Am die in Anspruch 1 angegebenen Bedeutungen besitzt, umsetzt, wobei man die Reaktion am Rückfluß in einem geeigneten Lösungsmittel durchführt zur Bildung des gewünschten Derivats, welches man gewünschtenfalls mit einer geeigneten organischen oder anorganischen Säure zur Bildung eines pharmazeutisch annehmbaren Salzes umsetzen kann.

**10.** Verfahren zur Herstellung der Aminoalkoxyphenylderivate nach Anspruch 1, worin A eine gegebenenfalls substituierte 2-Hydroxypropylengruppe und Beine -S- oder $-SO_2$-Gruppe bedeuten, **dadurch gekennzeichnet**, daß man ein Oxiranylmethoxyderivat der allgemeinen Formel:

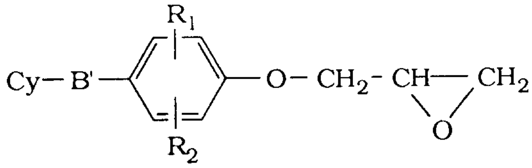

(57)

in der B' eine -S- oder $-SO_2$-Gruppe darstellt und Cy, $R_1$ und $R_2$ die in Anspruch 1 angegebenen Bedeutungen besitzen, am Rückfluß mit einen, Amin der allgemeinen Formel:

H - Am (3)

in der Am die in Anspruch 1 angegebenen Bedeutungen besitzt, umsetzt, und zwar in einem polaren Lösungsmittel oder in einem Überschuß des genannten Amins, so daß man:

- das gewünschte Derivat in Form der freien Base erhält, worin A eine 2-Hydroxypropylengruppe darstellt.
- ein Aminoalkoxyphenylderivat erhält, welches man mit einem Alkylhalogenid mit 1 bis 4 Kohlenstoffatomen in Gegenwart einer starken Base umsetzen kann, so daß man das gewünschte Derivat in Form der freien Base erhält, in dem A eine 2-Hydroxypropylengruppe darstellt, in der die Hydroxylgruppe durch eine Alkylgruppe mit 1 bis 4 Kohlenstoffatomen substituiert ist,

welches in dieser Weise erhaltene Aminoalkoxyphenylderivat gewünschtenfalls mit einer organischen oder anorganischen Säure zur Bildung eines pharmazeutisch annehmbaren Salzes umgesetzt werden kann.

**11.** Verfahren zur Herstellung der Aminoalkoxyphenylderivate nach Anspruch 1, worin B eine -SO-Gruppe darstellt, **dadurch gekennzeichnet**, daß man ein Sulfid der allgemeinen Formel:

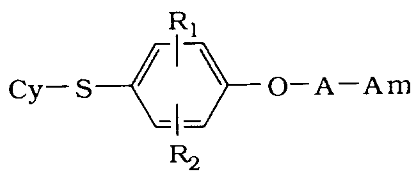

in der Cy, $R_1$, $R_2$, A und Am die in Anspruch 1 angegebenen Bedeutungen besitzen, welches Sulfid in Form der freien Base oder des Salzes vorliegt, mit einem Oxidationsmittel behandelt zur Bildung des gewünschten Derivats in Form der freien Base oder des Salzes, welches man mit einem basischen Mittel behandelt zur Bildung des gewünschten Derivats in Form der freien Base, welche in dieser Weise erhaltene freie Base gewünschtenfalls mit einer organischen oder anorganischen Säure zur Bildung eines pharmazeutisch annehmbaren Salzes dieses Derivats umgesetzt werden kann.

**12.** Verfahren nach Anspruch 11, **dadurch gekennzeichnet**, daß das Oxidationsmittel aus Natriumperiodat, Kaliumpermanganat und 3-Chlor-perbenzoesäure ausgewählt wird.

**13.** Pharmazeutische oder veterinärmedizinische Zubereitungen enthaltend als Wirkstoff mindestens ein Aminoalkoxyphenylderivat nach einem der Ansprüche 1 bis 6 in Kombination mit einem pharmazeutischen Trägermaterial oder einem geeigneten Bindemittel.

**14.** Pharmazeutische oder veterinärmedizinische Zubereitungen enthaltend als Wirkstoff mindestens ein Aminoalkoxyphenylderivat nach Anspruch 7 in Kombination mit einem pharmazeutischen Trägermaterial oder einem geeigneten Bindemittel.

**15.** Pharmazeutische oder veterinärmedizinische Zubereitungen nach einem der Ansprüche 13 oder 14 zur Behandlung von pathologischen Syndromen des kardiovaskulären Systems, enthaltend 50 mg bis 500 mg des Wirkstoffs.

**16.** Verwendung mindestens eines Aminoalkoxyphenylderivats nach einem der Ansprüche 1 bis 7 zur Herstellung eines Arzneimittels.

**17.** Verwendung mindestens eines Aminoalkoxyphenylderivats nach einem der Ansprüche 1 bis 7 zur Herstellung eines Arzneimittels zur Behandlung von pathologischen Syndromen des kardiovaskulären Systems.

**18.** Verwendung mindestens eines Aminoalkoxyphenylderivats nach einem der Ansprüche 1 bis 7 zur Herstellung eines Arzneimittels zur Behandlung von pathologischen Zuständen der Augen.

19. Verwendung mindestens eines Aminoalkoxyphenylderivats nach einem der Ansprüche 1 bis 7 zur Herstellung eines Arzneimittels zur Steigerung der Wirkung von Antikrebsmitteln.

**Patentansprüche für folgenden Vertragsstaat : ES**

1. Verfahren zur Herstellung von Aminoalkoxyphenylderivaten der allgemeinen Formel:

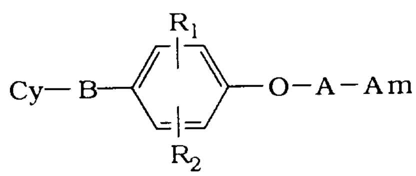

(1)

sowie deren pharmazeutisch annehmbare Salze, worin:

B eine -S-, -SO- oder -$SO_2$-Gruppe,

$R_1$ und $R_2$, die gleichartig oder verschieden sind, jeweils Wasserstoff, eine Methyl- oder Ethylgruppe oder ein Halogenatom, wie ein Chlor-, Brom- oder Iodatom,

A eine geradkettige oder verzweigte Alkylengruppe mit 2 bis 5 Kohlenstoffatomen oder die 2-Hydroxy-propylengruppe, worin die Hydroxylgruppe gegebenenfalls durch eine Alkylgruppe mit bis zu vier Kohlenstoffatomen substituiert ist,

Am eine Gruppe:

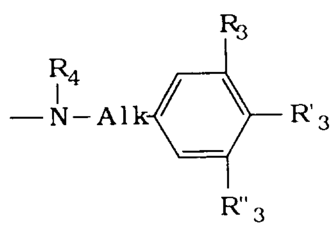

oder

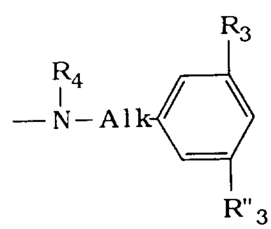

in denen $R_3$, $R'_3$ und $R''_3$, die gleichartig oder verschieden sind, jeweils ein Halogenatom, wie ein Chlor- oder ein Bromatom, eine Alkylgruppe mit bis zu vier Kohlenstoffatomen oder eine Alkoxygruppe mit bis zu vier Kohlenstoffatomen oder eine Benzyloxygruppe,

$R_4$ ein Wasserstoffatom oder eine Alkylgruppe mit bis zu acht Kohlenstoffatomen,

Alk eine Einfachbindung oder eine geradkettige oder verzweigte Alkylengruppe mit 1 bis 5 Kohlenstoffatomen darstellen und

Cy eine Gruppe der Formeln

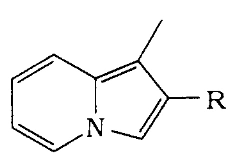

oder

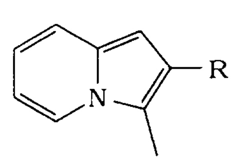

worin

R ein Wasserstoffatom, eine Alkylgruppe mit bis zu acht Kohlenstoffatomen, eine $C_3$-$C_6$-Cycloalkylgruppe, eine Benzylgruppe oder eine Phenylgruppe, die gegebenenfalls durch einen oder mehrere Substituenten substituiert ist, die gleichartig oder verschieden sein können und aus Halogenatomen oder Alkylgruppen mit bis zu vier Kohlenstoffatomen, Alkoxygruppen mit bis zu vier Kohlenstoffatomen und Nitrogruppen ausgewählt sind, substituiert sein können, darstellt, bedeuten,

**dadurch gekennzeichnet**, daß man

A/ wenn A eine Alkylengruppe und B eine -S- oder $-SO_2$-Gruppe bedeuten, man

- entweder ein 4-Alkoxyphenylderivat der allgemeinen Formel:

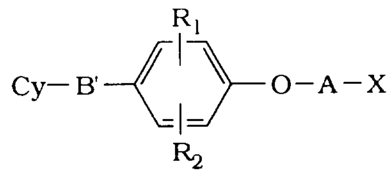

(2)

in der B' eine -S- oder $-SO_2$-Gruppe darstellt, Cy, $R_1$ und $R_2$ die oben angegebenen Bedeutungen besitzen, A die oben angegebenen Bedeutungen besitzt und X ein Halogenatom oder eine Alkylsulfonyloxygruppe mit 1 bis 4 Kohlenstoffatomen oder eine Arylsulfonyloxygruppe mit 6 bis 10 Kohlenstoffatmen bedeutet,
in Gegenwart eines Säureakzeptors in einem polaren oder nichtpolaren Lösungsmittel bei einer Temperatur zwischen Raumtemperatur und der Rückflußtemperatur mit einem Amin der allgemeinen Formel:

H - Am (3)

in der Am die oben angegebenen Bedeutungen besitzt` zur Bildung des gewünschten Derivats in Form der freien Base,

- oder ein 4-Hydroxyphenylderivat der allgemeinen Formel:

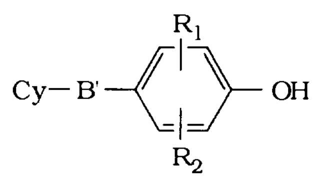

(4)

in der B' eine -S- oder $-SO_2$-Gruppe darstellt und Cy, $R_1$ und $R_2$ die oben angegebenen Bedeutungen besitzen, in Gegenwart eines basischen Mittels mit einer Verbindung der allgemeinen Formel:

X - A - Am (52)

in der X ein Halogenatom oder eine Alkylsulfonyloxygruppe mit 1 bis 4 Kohlenstoffatomen oder eine Arylsulfonyloxygruppe mit 6 bis 10 Kohlenstoffatomen und A eine Alkylengruppe darstellen und Am die oben angegebenen Bedeutungen besitzt, umsetzt, wobei die Reaktion am Rückfluß in einem geeigneten Lösungsmittel durchgeführt wird zur Bildung des gewünschten Derivats in Form der freien Base;

B/ wenn A eine gegebenenfalls substituierte 2-Hydroxypropylengruppe und B eine -S- oder $-SO_2$-Gruppe bedeuten, man ein Oxiranylmethoxyderivat der allgemeinen Formel:

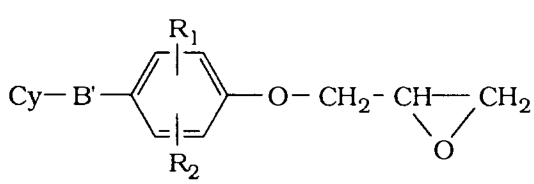

(57)

in der B' eine -S- oder $-SO_2$-Gruppe darstellt und Cy, $R_1$ und $R_2$ die oben angegebenen Bedeutungen besitzen, am Rückfluß mit einem Amin der allgemeinen Formel:

H - Am (3)

in der Am die oben angegebenen Bedeutungen besitzt, in einem polaren Lösungsmittel oder in einem Überschuß des genannten Amins umsetzt zur Bildung:

- des gewünschten Derivats in Form der freien Base, worin A eine 2-Hydroxypropylengruppe darstellt,
- eines Aminoalkoxyphenylderivats, welches man mit einem Alkylhalogenid mit 1 bis 4 Kohlenstoffatomen in Gegenwart einer starken Base umsetzen kann zur Bildung des gewünschten Derivats in Form der freien Base, worin A eine 2-Hydroxypropylengruppe darstellt, in der die Hydroxylgruppe durch eine Alkylgruppe mit 1 bis 4 Kohlenstoffatomen substituiert ist, und

C/ wenn B eine -SO-Gruppe bedeutet, man ein Sulfid der allgemeinen Formel:

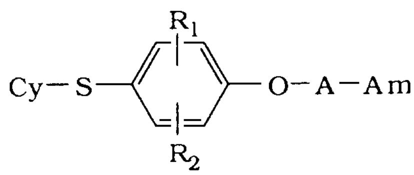

in der Cy, $R_1$, $R_2$, A und Am die oben angegebenen Bedeutungen besitzen, welches Sulfid in Form der freien Base oder des Salzes vorliegt, mit einem Oxidationsmittel behandelt zur Bildung des gewünschten Derivats in Form der freien Base oder des Salzes, welches man mit einem basischen Mittel behandelt zur Bildung des gewünschten Derivats in Form der freien Base:

welche erhaltene freie Base gewünschtenfalls mit einer organischen oder anorganischen Säure zur Bildung eines pharmazeutisch annehmbaren Salzes des Derivats der Formel (1) umgesetzt werden kann.

2. Verfahren nach Anspruch 1, **dadurch gekennzeichnet**, daß das Oxidationsmittel aus Natriumperiodat, Kaliumpermanganat und 3-Chlor-perbenzoesäure ausgewählt ist.

3. Verfahren nach Anspruch 1, **dadurch gekennzeichnet**, daß man Aminoalkoxyphenylderivate der Formel (1) herstellt, in der B eine -$SO_2$-Gruppe darstellt.

4. Verfahren nach Anspruch 1, **dadurch gekennzeichnet**, daß man Aminoalkoxyphenylderivate der Formel (1) herstellt, in der $R_1$ und $R_2$ jeweils Wasserstoff bedeuten.

5. Verfahren nach Anspruch 1, **dadurch gekennzeichnet**, daß man Aminoalkoxyphenylderivate der Formel (1) herstellt, in der $R_3$, $R'_3$ und $R''_3$ Methoxygruppen bedeuten.

6. Verfahren nach Anspruch 1, **dadurch gekennzeichnet**, daß man Aminoalkoxyphenylderivate der Formel (1) herstellt, in der R eine Isopropyl- oder Cyclopropylgruppe darstellt.

7. Verfahren nach Anspruch 1, **dadurch gekennzeichnet**, daß man Aminoalkoxyphenylderivate der Formel (1) herstellt, in der das pharmazeutisch annehmbare Salz das Oxalat, Hydrochlorid oder Methansulfonat ist.

8. Verfahren nach Anspruch 1, **dadurch gekennzeichnet**, daß man die Aminoalkoxyphenylderivate der Formel (1) herstellt, die ausgewählt sind aus:
1-[(3-[N-Methyl-N-(3,4,5-trimethoxy-$\beta$-phenethyl)-amino]-propyl}-4-oxy-benzolsulfonyl]-2-isopropyl-indolizin,
1-[(3-[N-Methyl-N-(3,4-dimethoxy-5-methyl-$\beta$-phenethyl)-amino]-propyl}-4-oxy-benzolsulfonyl]-2-isopropyl-indolizin,
1-[(3-[N-(3,5-Dimethoxy-$\beta$-phenethyl)-amino)-propyl)-4-oxy-benzolsulfonyl]-2-isopropyl-indolizin
und dessen pharmazeutisch annehmbaren Salzen.

9. Verfahren zur Herstellung von pharmazeutischen oder veterinärmedizinisehen Zubereitungen, **dadurch gekennzeichnet**, daß man zu mindestens einem nach einem der vorhergehenden Ansprüche erhaltenen Aminoalkoxyphenylderivat ein geeignetes pharmazeutisches Trägermaterial oder Bindemittel zusetzt.

10. Verfahren zur Herstellung von pharmazeutischen Zubereitungen nach Anspruch 9 für die Behandlung von pathologischen Syndromen des kardiovaskulären Systems, enthaltend 50 mg bis 500 mg des Wirkstoffs.

11. Verfahren zur Herstellung von pharmazeutischen Zubereitungen nach Anspruch 9 zur Behandlung von pathologischen Zuständen der Augen.

12. Verfahren zur Herstellung von pharmazeutischen Zubereitungen nach Anspruch 9 zur Förderung der Wirksamkeit von Antikrebsmitteln.

**Patentansprüche für folgenden Vertragsstaat : GR**

1. Aminoalkoxyphenylderivate der allgemeinen Formel:

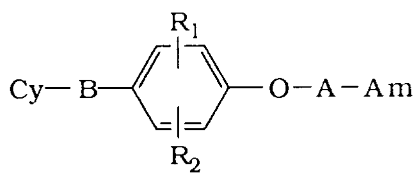

(1)

sowie deren pharmazeutisch annehmbare Salze, worin:

B eine -S-, -SO- oder $-SO_2-$Gruppe,

$R_1$ und $R_2$, die gleichartig oder verschieden sind, jeweils Wasserstoff, eine Methyl- oder Ethylgruppe oder ein Halogenatom, wie ein Chlor-, Brom- oder Iodatom,

A eine geradkettige oder verzweigte Alkylengruppe mit 2 bis 5 Kohlenstoffatomen oder die 2-Hydroxy-propylengruppe, worin die Hydroxylgruppe gegebenenfalls durch eine Alkylgruppe mit bis zu vier Kohlenstoffatomen substituiert ist,

Am eine Gruppe:

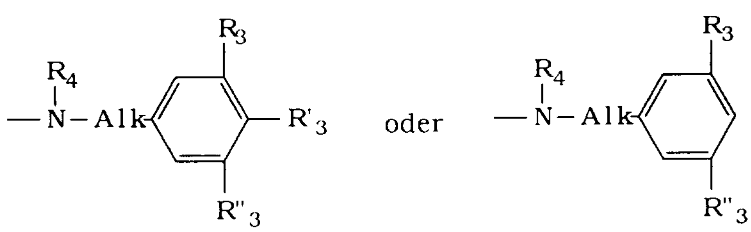

in denen $R_3$, $R'_3$ und $R''_3$, die gleichartig oder verschieden sind, jeweils ein Halogenatom, wie ein Chlor- oder ein Bromatom, eine Alkylgruppe mit bis zu vier Kohlenstoffatomen oder eine Alkoxygruppe mit bis zu vier Kohlenstoffatomen oder eine Benzyloxygruppe,

$R_4$ ein Wasserstoffatom oder eine Alkylgruppe mit bis zu acht Kohlenstoffatomen,

Alk eine Einfachbindung oder eine geradkettige oder verzweigte Alkylengruppe mit 1 bis 5 Kohlenstoffatomen darstellen und

Cy eine Gruppe der Formeln

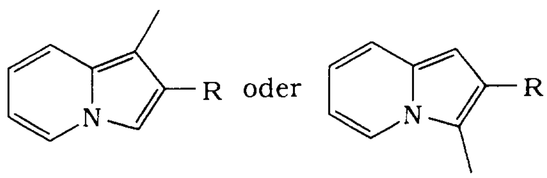

worin
R ein Wasserstoffatom, eine Alkylgruppe mit bis zu acht Kohlenstoffatomen, eine $C_3$-$C_6$-Cycloalkylgruppe, eine Benzylgruppe oder eine Phenylgruppe, die gegebenenfalls durch einen oder mehrere Substituenten substituiert ist, die gleichartig oder verschieden sein können und aus Halogenatomen oder Alkylgruppen mit bis zu vier Kohlenstoffatomen, Alkoxygruppen mit bis zu vier Kohlenstoffatomen und Nitrogruppen ausgewählt sind, substituiert sein können, darstellt, bedeuten.

**2.** Aminoalkoxyphenylderivate nach Anspruch 1, worin B eine -$SO_2$-Gruppe bedeutet.

**3.** Aminoalkoxyphenylderivate nach Anspruch 1, worin $R_1$ und $R_2$ jeweils Wasserstoff bedeuten.

**4.** Aminoalkoxyphenylderivate nach Anspruch 1, worin $R_3$, $R'_3$ und $R''_3$ Methoxygruppen bedeuten.

**5.** Aminoalkoxyphenylderivate nach Anspruch 1, worin R die Isopropyl- oder Cyclopropylgruppe bedeutet.

**6.** Aminoalkoxyphenylderivate nach Anspruch 1, worin das pharmazeutisch annehmbare Salz das Oxalat, das Hydrochlorid oder das Methansulfonat ist.

**7.** Aminoalkoxyphenylderivate nach Anspruch 1 ausgewählt aus:
1-[{3-[N-Methyl-N-(3,4,5-trimethoxy-β-phenethyl)-amino]-propyl}-4-oxy-benzolsulfonyl]-2-isopropyl-indolizin,
1-[{3-[N-Methyl-N-(3,4-dimethoxy-5-methyl-β-phenethyl)-amino]-propyl}-4-oxy-benzolsulfonyl]-2-isopropyl-indolizin,
1-[(3-[N-(3,5-Dimethoxy-β-phenethyl)-amino]-propyl}-4-oxybenzolsulfonyl]-2-isopropyl-indolizin
und dessen pharmazeutisch annehmbaren Salzen.

**8.** Verfahren zur Herstellung der Aminoalkoxyphenylderivate nach Anspruch 1, worin A eine Alkylengruppe und B eine -S- oder -$SO_2$-Gruppe bedeuten, **dadurch gekennzeichnet**, daß man ein 4-Alkoxyphenylderivat der allgemeinen Formel:

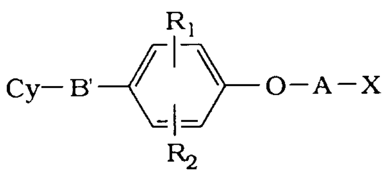
(2)

in der B' eine -S- oder -$SO_2$-Gruppe darstellt, Cy, $R_1$ und $R_2$ die in Anspruch 1 angegebenen Bedeutungen besitzen, A die oben angegebenen Bedeutungen besitzt und X ein Halogenatom oder eine Alkylsulfonyloxygruppe mit 1 bis 4 Kohlenstoffatomen oder eine Arylsulfonyloxygruppe mit 6 bis 10 Kohlenstoffatmen darstellt,
in Gegenwart eines Säureakzeptors in einem polaren oder nichtpolaren Lösungsmittel bei einer Temperatur zwischen Raumtemperatur und der Rückflußtemperatur mit einem Amin der allgemeinen Formel:

H - Am (3)

in der Am die in Anspruch 1 angegebenen Bedeutungen besitzt, kondensiert zur Bildung des

gewünschten Derivats, welches man gewünschtenfalls mit einer organischen oder anorganischen Säure zur Bildung eines pharmazeutisch annehmbaren Salzes dieses Derivats umsetzt.

**9.** Verfahren zur Herstellung der Aminoalkoxyphenylderivate nach Anspruch 1, worin A eine Alkylengruppe und B eine -S- oder -$SO_2$-Gruppe bedeuten, **dadurch gekennzeichnet**, daß man ein 4-Hydroxyphenylderivat der allgemeinen Formel:

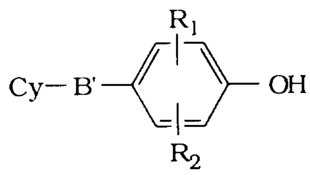

(4)

in der B' eine -S- oder -$SO_2$-Gruppe darstellt und Cy, $R_1$ und $R_2$ die in Anspruch 1 angegebenen Bedeutungen besitzen, in Gegenwart eines basischen Mittels mit einer Verbindung der allgemeinen Formel:

X - A - Am (52)

in der X ein Halogenatom oder eine Alkylsulfonyloxygruppe mit 1 bis 4 Kohlenstoffatomen oder eine Arylsulfonyloxygruppe mit 6 bis 10 Kohlenstoffatomen und A eine Alkylengruppe darstellen und Am die in Anspruch 1 angegebenen Bedeutungen besitzt, umsetzt, wobei man die Reaktion am Rückfluß in einem geeigneten Lösungsmittel durchführt zur Bildung des gewünschten Derivats, welches man gewünschtenfalls mit einer geeigneten organischen oder anorganischen Säure zur Bildung eines pharmazeutisch annehmbaren Salzes umsetzen kann.

**10.** Verfahren zur Herstellung der Aminoalkoxyphenylderivate nach Anspruch 1, worin A eine gegebenenfalls substituierte 2-Hydroxypropylengruppe und Beine -S- oder -$SO_2$-Gruppe bedeuten, **dadurch gekennzeichnet**, daß man ein Oxiranylmethoxyderivat der allgemeinen Formel:

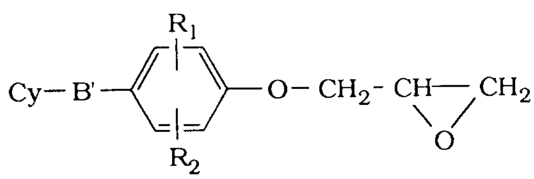

(57)

in der B' eine -S- oder -$SO_2$-Gruppe darstellt und Cy, $R_1$ und $R_2$ die in Anspruch 1 angegebenen Bedeutungen besitzen, am Rückfluß mit einem Amin der allgemeinen Formel:

H - Am (3)

in der Am die in Anspruch 1 angegebenen Bedeutungen besitzt, umsetzt, und zwar in einem polaren Lösungsmittel oder in einem Überschuß des genannten Amins, so daß man:

- das gewünschte Derivat in Form der freien Base erhält, worin A eine 2-Hydroxypropylengruppe darstellt,
- ein Aminoalkoxyphenylderivat erhält, welches man mit einem Alkylhalogenid mit 1 bis 4 Kohlenstoffatomen in Gegenwart einer starken Base umsetzen kann, so daß man das gewünschte Derivat in Form der freien Base erhält, in dem A eine 2-Hydroxypropylengruppe darstellt, in der die Hydroxylgruppe durch eine Alkylgruppe mit 1 bis 4 Kohlenstoffatomen substituiert ist,

welches in dieser Weise erhaltene Aminoalkoxyphenylderivat gewünschtenfalls mit einer organischen oder anorganischen Säure zur Bildung eines pharmazeutisch annehmbaren Salzes umgesetzt werden kann.

**11.** Verfahren zur Herstellung der Aminoalkoxyphenylderivate nach Anspruch 1, worin B eine -SO-Gruppe darstellt, **dadurch gekennzeichnet**, daß man ein Sulfid der allgemeinen Formel:

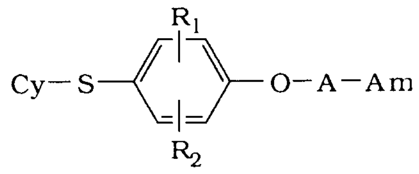

in der Cy, $R_1$, $R_2$, A und Am die in Anspruch 1 angegebenen Bedeutungen besitzen, welches Sulfid in Form der freien Base oder des Salzes vorliegt, mit einem Oxidationsmittel behandelt zur Bildung des gewünschten Derivats in Form der freien Base oder des Salzes, welches man mit einem basischen Mittel behandelt zur Bildung des gewünschten Derivats in Form der freien Base, welche in dieser Weise erhaltene freie Base gewünschtenfalls mit einer organischen oder anorganischen Säure zur Bildung eines pharmazeutisch annehmbaren Salzes dieses Derivats umgesetzt werden kann.

**12.** Verfahren nach Anspruch 11, **dadurch gekennzeichnet**, daß das Oxidationsmittel aus Natriumperiodat, Kaliumpermanganat und 3-Chlor-perbenzoesäure ausgewählt wird.

**13.** Verfahren zur Herstellung von pharmazeutischen oder veterinärmedizinischen Zubereitungen, **dadurch gekennzeichnet**, daß man zu mindestens einem Aminoalkoxyphenylderivat nach einem der Ansprüche 1 bis 6 ein geeignetes pharmazeutisches Trägermaterial oder Bindemittel zusetzt.

**14.** Verfahren zur Herstellung von pharmazeutischen oder veterinärmedizinischen Zubereitungen, **dadurch gekennzeichnet**, daß man zu mindestens einem Aminoalkoxyphenylderivat nach Anspruch 7 ein geeignetes pharmazeutisches Trägermaterial oder Bindemittel zusetzt.

**15.** Verfahren zur Herstellung von pharmazeutischen oder veterinärmedizinischen Zubereitungen nach einem der Ansprüche 13 oder 14 für die Behandlung von pathologischen Syndromen des kardiovaskulären Systems, enthaltend 50 mg bis 500 mg des Wirkstoffs.

**16.** Verfahren zur Herstellung von pharmazeutischen oder veterinärmedizinisehen Zubereitungen nach einem der Ansprüche 13 oder 14 für die Behandlung von pathologischen Zuständen der Augen.

**17.** Verfahren zur Herstellung von pharmazeutischen oder veterinärmedizinischen Zubereitungen nach einem der Ansprüche 13 oder 14 für die Verstärkung der Wirkung von Antikrebsmitteln.